# EUROPEAN PATENT APPLICATION

(11) **EP 4 166 670 A1**
(43) Date of publication of application: **19.04.2023**
(21) Application number: 21203093.6
(22) Date of filing: 18.10.2021
(51) Int. Cl.: C12N 15/82

(54) **PLANT-TAG-BASED WEEDING CONTROL**

(71) Applicant: KWS SAAT SE & Co. KGaA, 37574 Einbeck (DE)
(72) Inventor: Meldau, Stefan, 37077 Göttingen (DE); Langenbach, Caspar, 4728 Hergenrath (BE); Kins, Lisa, 26121 Oldenburg (DE); Wildhagen, Hanna, 37075 Göttingen (DE); Bauer, Christoph, 37574 Einbeck (DE)
(74) Representative: Eisenführ Speiser

(57) **Abstract**

The present invention relates to means and methods for precise differentiation of crop plants and weeds that on can be used by automated weeding platforms. In particular, the invention relates to a method of labelling crop plants with a color tag that, when active, gives rise to a color signal detectable by a cognate sensor. Further, it relates to a method of distinguishing crop plants from weeds using color tags and weeding platforms. Finally, it also relates to the use of a color tag for automated robot assisted weeding.

## Description

### Technical Field

The present invention provides means and methods for precise differentiation of crop plants and weeds that on can be used by automated weeding platforms. In particular, the invention relates to a method of labelling crop plants with a color tag that, when active, gives rise to a color signal detectable by a cognate sensor. Further, it relates to a method of distinguishing crop plants from weeds using color tags and weeding platforms. Finally, it also relates to the use of a color tag for automated robot assisted weeding.

### Background

The future is turning into a world where agriculture and farming is conducted by mobile platforms. Especially the development of many different weeding robots points out that the main problem for these sensor-based platforms is to distinguish reliably between weed and crop leaves. This is caused by the fact that the shape of leaves can generally vary substantially in young plants. Weeding is particularly important in the first weeks of plant development because crop plants and weeds compete for the abiotic resources light, water and nutrients. Therefore, it is essential that weeding is conducted in these first weeks so that crop plants can grow well in this critical timeframe, leaving weeds very low chances of competing once the crop plants have grown past their early developmental stages. In an agricultural industry that relies more and more on robotics and automation for rapid and cost-efficient production, it is a particular conundrum that weeding has to occur in the very early stage after germination, while it is exactly this time period that is especially problematic for sensor-based detection of characteristic leaf shapes.

The work in the area of robotic weed management and control demonstrates the potential of automated weeding technology to reduce the efforts and costs significantly by treating fields on a much-reduced scale than conventional agriculture. So far, a large amount of work has been directed to the weed/crop plant perception problem (cf. Steward, et al., The use of agricultural robots in weed management and control, Robotics and automation for improving agriculture, Advanced Machinery Engineering and Manufacturing Systems, 2019, doi: 10.19103/AS.2019.0056.13). As disclosed in Steward et al., *supra*, this perception problem has been particularly challenging given the semi-controlled nature of agricultural fields. Variability in lighting, density and species of weed plants, and occlusion of mixtures of plants are some of the main challenges associated with perception systems. Much progress has been reported using combinations of sensors and newer artificial intelligence (Al) approaches and a variety of robotic weed control mechanisms have been explored for both chemical and mechanical weed control approaches. In many cases, mechanical weeders show potential to be efficacious under some conditions, but weed control performance studies have been limited. Further, weeders that have machinery behavior technologies included in them are limited and current robotic weeder examples are limited in being able to achieve goals in the context of variation and uncertainty. For example, there are no reported examples of robotic weeders being able to change tools or adjust tool control due to changing soil conditions. Color cameras, including those measuring near-infrared (NIR) light and cognate sensors as well as algorithms using visible color reflectance differences are available. Yet in practice these techniques so far only make use of colors naturally occurring during a certain developmental stage, particularly by detecting chlorophyll in the leaves of crop plants etc. This differentiation is, however, still challenging, especially in ill-controlled, variable surroundings.

So far, it is thus a tremendous drawback of automated weeding platforms that these are not able to discriminate a seed/seedling in a very early developmental stage from a weed with sufficient precision. Particularly in such an early developmental stage, weed control would be needed to help the seedling to outgrow in an environment, where weeds compete for nutrients. The problem is that there is no described characteristic that would allow a weeding platform to distinguish reliably between crop plants and weeds in such an early developmental stage. The invention described herein addresses this problem and allows the detection of unwanted weeds by a different detection method. This new detection method is based on the labelling of crop plants resulting in a clearly distinguishable optical signal, thereby providing the possibility of a unique identification of a crop plant. In the early stage of plant growth, such a label is present in the cotyledon and/or hypocotyl or otherwise in the canopy, generating a detectable signal. Unwanted plants, such as weeds, do not emit such a signal, which identifies them as a potential weeding target. Based on this accurate classification, a precise weeding via sensor-based platforms is much easier to realize and that with significantly less sources of error than by conventional methods. Due to the improved targeted elimination of undesirable weeds the use herbicides in crop plant production can be reduced.

The present invention thus tries to address the above need for a system that allows reliable differentiation of crops and weed in young plants without affecting the harvested plant or its harvested products. This invention will overall improve the upcoming technology of weeding platforms. The innovative character can be seen in the change from analyzing leaf shape to perceiving precise signals of stained crop plants. Current weeding robots are using RGB-cameras, this could be replaced by multispectral or hyperspectral imaging sensors for detecting the color. An advantage of all the labelling methods according to the present invention is that already the seeds of plants can comprise the color tag and the subsequently growing seedling will show a distinct color signal without further processing steps.

Such a system might even be complemented by artificial intelligence evaluation processes. Sensor-based platforms already learn how to differentiate leaf shapes of weeds from crop plants leaves by artificial intelligence but due to the great variety of leaf shapes, this is not sufficiently precise so far. In contrast, the differentiation of the desired crop plants from other plants by labelling with a color tag offers a much more preferable basis for AI evaluation processes.

While there are studies addressing the transport of the fluorescent coumarin β-glucoside esculin into yeast cells (Gora et al. 2012, Plant Methods, 8:13) or protoplasts (Rottman et al. 2018, Front Plant Sci. Oct 24;9:1551), these studies focused entirely on the measurement of transport and the characterization of the sucrose transporters without offering any insight into the possibility of labelling plants. Natalio et. al. (2017 Science 357, 1118-1122) could show that the fluorescent compound 6-carboxyfluorescein-glucose (6CF-Glc) and the magnetic Glucose- 1,4,7,10-tetraazacyclododecane-1,4,7,10-tetraacetic acid dysprosium complex (Glc-DOTA-Dy(III)) can be integrated into cotton fibers of *Gossypium hirsutum.* These teachings still do not offer any insight into labelling whole seedlings and, further, they address the integration of compounds into the final product of crop plants with the specific intention to alter the properties of said product. Thus, they offer no viable basis for a labelling of crop plants in the early growth phase.

Therefore, there is an urgent need to provide a basis for automated weeding platforms to distinguish reliably between the desired crop plants and other, unwanted plants in a manner that does not alter the harvested product.

### Summary of Invention

To address the need for a reliable distinction between crop plants and weeds in the early growth phase, the present invention provides color tags, which, when active, can be recognized by a sensor, to allow precise weeding by automated weeding platforms.

In a first aspect the present invention relates to a method for labelling a plant, plant cell, tissue, organ, seed, or material with at least one color tag active at least during an early growth stage of a plant and allowing the discrimination of desired plants from undesired weeds by means of color signals, wherein the at least one color tag is detectable by at least one cognate sensor, the method comprising: (i) providing at least one plant cell, or at least one plant, tissue, organ, seed, or material comprising the same; (ii) introducing or inducing at least one color tag into/in the at least one plant cell, or into the at least one plant, tissue, organ, seed, or material comprising the same; (iii) optionally: selecting at least one plant cell, or at least one plant, tissue, organ, seed, or material comprising the same, wherein the selection is based on the successful integration of the color tag; (iv) obtaining at least one plant cell, or at least one plant, tissue, organ, seed, or material comprising the same, that comprises the color tag, so that the color tag is active during the early growth stage of a plant; and (v) optionally: seeding the plant seed and discriminating the desired plants comprising an active color tag from undesired weeds; wherein the color tag is introduced in a transient way, or wherein the color tag is a natural color tag, or any combination thereof.

In a preferred embodiment of the first aspect of the present invention, the color tag is introduced by labelling a plant seed with a chemical color tag, or by introducing a genomic color tag, or any combination thereof.

In preferred embodiments of the first aspect of the present invention the early growth stage is the time period up to the 4-leaf-stage.

In one embodiment of the first aspect of to the present invention, the color tag is a chemical color tag, preferably wherein the chemical color tag consists of or comprises a dye being selected from the group of a coumarin, including scopoletin or esculetin, more preferably wherein the chemical color tag consists of or comprises a dye being coupled to glucose, including esculin or scopolin.

In a further embodiment of the first aspect according to the present invention the color tag is a genomic color tag introduced by a genome modification system by (a) introducing at least one insertion nucleotide sequence, wherein the expression of said insertion nucleotide sequence causes an altered level of at least one compound detectable by at least one cognate sensor, wherein the at least one insertion nucleotide sequence is a plant endogenous sequence, or by (b) modifying or introducing at least one regulatory region that causes an altered expression of an endogenous nucleotide sequence, wherein the altered expression of said endogenous nucleotide sequence causes an altered level of at least one compound detectable by at least one cognate sensor, or any combination of (a) and (b).

In certain embodiments of the first aspect of the present invention the genome modification system comprises at least one site-directed nuclease, nickase or an inactivated nuclease, preferably a nucleic acid guided nuclease, nickase or an inactivated nuclease, or a sequence encoding the same, and optionally at least one guide molecule, or a sequence encoding the same.

In certain embodiments of the first aspect according to the present invention, the at least one cognate sensor is an image sensor, optionally included in a camera, wherein the image sensor is an RGB sensor, a multispectral sensor, a hyperspectral sensor, a fluorescence sensor, or a monochrome sensor, or a combination of at least two sensors, wherein the at least one cognate sensor may optionally comprise an irradiation unit for irradiating at least one plant, plant cell, tissue, organ, seed, or material with light, wherein said modulated irradiation source is preferably used in combination with the image sensor.

In certain embodiments of the first aspect according to the present invention, the at least one undesired weed is selected from the group consisting of *Alopecures myosuroides, Acalypha australis L., Amaranthus retroflexus, Agropyron repens (L) Beauv., Agrostis gigantea Roth. Fl. Germ, Brassica napus, Amaranthus blitoide, Amaranthus lividus Loisel, Amaranthus palmerii, Amaranthus patulus bertoloni, Amaranthus powellii, Capsella bursa-pastoris, Ambrosia artemisiifolia, Ambrosia trifida, Amethystea caerulea L., Anthemis arvensis L., Anthemis cotula L., Artemisia scoparia, Artemisia sieversiana, Chenopodium album, Fumaria officinalis, Galeopsis tetrahit, Barbarea vulgaris R.Br., Bidens tripartite, Galium aparine, Calystegia hederacea Wall, Calystegia sepium, Geranium spp., Cenchrus echinatus, Centaurea cyanus, Lamium purpureum, Chenopodium glaucum L., Chenopodium serotinum L., Matricaria camomilla, Cirsuim vulgare, Commelina communis L., Conium maculatum, Matricaria inodora, Cuscuta campestris (pentago-na), Cuscuta pentagona Engelm., Cyperus esculentus, Datura stramonium L., Digitaria sanguinalis, Digitaria violascens Link, Mercurialis annual, Echinochloa phyllopogon Stapf, Eleusine inica(L) Gaertn., Elsholtzia ciliata (Thunb.) Hylander, Poa Annua, Polygonum aviculare, Erigeron annuus L., Erigeron canadensis L., Erysimum cheiranthoides L., Fallopia japonica, Polygonum convolvu-lus, Polygonum persicaria, Galinsoga parviflora, Senecio vulgaris, Solanum nigrum, Helianthus annuus, Hibiscus trionum, Hordeum vulgare, Iva annua, Kochia scoparia, Stellaria media, Thlaspi arvense, Veronica arvensis, Viola arvensis, Abutilon theophrasti, Aethusa cynapium, Myosoton aquaticum (L.) Moench, Oxalis corniculata L., Panicum capillare, Panicum dichotomiflorum, Persicaria convolvulus L., Persicaria lapathifolia (L.) S.F.Gray, Persicaria longiseta (De Bruyn) Kitag., Persicaria nepalensis (Meisn.) H.Gross, Persicaria vulgaris Webb et Moq., Phragmites australis (Cav.) Trin. Ex Steud., Atriplex patula, Cirsium arvense (Cirsium Segetum* / *Cephalonoplos segetum), Echinochloa crus-galii, Polygonum bungeanum T., Elymus repens* / *Elytrigia repens, Polygonum lapatifolium, Polygonum pensylvaticum, Myosotis arvensis, Portulaca oleracea, Rhaponticum repens, Rorippa atrovirens Ohwi et Hara, Rorippa islandica (Oeder) Borlas, Rorippa sylvestris Besser, Rumex acetosella L., Rumex crispus L., Rumex japonicus Houtt., Rumex longifolius DC., Rumex obtusifolius L., Sagina japonica (Sw.) Ohwi, Salsola tragus, Salvia reflexa, Sonchus arvensis (brachyotus DC), Setaria glauca, Setaria viridis, Atriplex hastate, Avena fatua, Solanum ptycanthum, Solanum rostratum, Solanum sarrachoides, Convolvulus arvensis, Equisetum arvense, Sonchus brachyotis DC., Spergula arvensis L., Malva spp., Stellaria alsine Grimm var.undulata (Thunb.) Ohwi, Polygonum amphibium, Symphytum officinale L., Taraxacum (officinale and mongolicum), Sinapis arvensis, Solanum tuberosum, Stachys arvensis, Veronica hederifolia L., Veronica persica, Urtica urens, Xanthium strumarium (Xanthium sibiricum P.), Lepidium didymum,* or any combination thereof.

A second aspect the present invention relates to a plant, plant cell, tissue, organ, material or seed obtainable by a method according to the above aspects and embodiments, wherein the color tag is introduced by labelling the seed with a chemical color tag or by introducing or inducing a genomic color tag using a genome modification system.

In one embodiment of the second aspect of to the present invention the plant, plant cell, tissue, organ, or seed is selected from a plant originating from a genus selected from the group consisting of *Hordeum, Sorghum, Saccharum, Zea, Setaria, Oryza, Triticum, Secale, Triticale, Malus, Brachypodium, Aegilops, Daucus, Beta, Eucalyptus, Nicotiana, Solanum, Coffea, Vitis, Erythrante, Genlisea, Cucumis, Marus, Arabidopsis, Crucihimalaya, Cardamine, Lepidium, Capsella, Olmarabidopsis, Arabis, Brassica, Eruca, Raphanus, Citrus, Jatropha, Populus, Medicago, Cicer, Cajanus, Phaseolus, Glycine, Gossypium, Astragalus, Lotus, Torenia, Allium, Spinacia or Helianthus,* preferably, the plant or plant cell originates from a species selected from the group consisting of *Hordeum vulgare, Hordeum bulbusom, Sorghum bicolor, Saccharum officinarium, Zea spp., including Zea mays, Setaria italica, Oryza minuta, Oryza sativa, Oryza australiensis, Oryza alta, Triticum aestivum, Triticum durum, Secale cereale,* Triticale, *Malus domestica, Brachypodium distachyon, Hordeum marinum, Aegilops tauschii, Daucus glochidiatus, Beta spp.,* including *Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Allium tuberosum, Helianthus annuus, Helianthus tuberosus* and/or *Spinacia oleracea.*

In a third aspect the present invention relates to a method of distinguishing at least one plant comprising at least one active color tag from potentially present undesired weeds, wherein the method comprises the following steps: (i) providing at least one plant that comprises at least one active color tag as defined in any one of the preceding aspects and embodiments, and further providing at least one platform, preferably a mobile platform, more preferably an autonomous mobile platform, equipped with at least one cognate sensor as defined above; (ii) optionally: irradiating the at least one plant with light; (iii) capturing image data of the at least one plant comprising the at least one active color tag using the at least one cognate sensor; (iv) processing the image data (v) distinguishing at plant comprising the active color tag from potentially present undesired weeds.

In a preferred embodiment of the third aspect of the present invention, the method comprises the additional step of (vi) of eliminating potentially present undesired weeds.

In a fourth aspect the invention relates to a use of an active color tag comprised by at least one plant, plant cell, tissue, organ, seed, or material, preferably during an early growth stage up to the 4-leaf-stage, for automated robot assisted weeding.

In one embodiment of the fourth aspect of the present invention, the color tag is a chemical color tag, wherein the chemical color tag consists of or comprises a dye being selected from the group of a coumarin, including scopoletin or esculetin, more preferably wherein the chemical color tag consists of or comprises a dye being coupled to glucose, including esculin or scopolin.

In another embodiment of the fourth aspect of the present invention, the color tag is a genomic color tag introduced by a genome modification system by (a) introducing at least one insertion nucleotide sequence, wherein the expression of said insertion nucleotide sequence causes an altered level of at least one compound detectable by at least one cognate sensor, wherein the at least one insertion nucleotide sequence is a plant endogenous sequence, or by (b) modifying or introducing at least one regulatory region that causes an altered expression of an endogenous nucleotide sequence, wherein the altered expression of said endogenous nucleotide sequence causes an altered level of at least one compound detectable by at least one cognate sensor, or any combination of (a) and (b).

### Definitions

A "base editor" as used herein refers to a protein or a fragment thereof having the same catalytic activity as the protein it is derived from, which protein or fragment thereof, alone or when provided as molecular complex, referred to as base editing complex herein, has the capacity to mediate a targeted base modification, i.e., the conversion of a base of interest resulting in a point mutation of interest which in turn can result in a targeted mutation, if the base conversion does not cause a silent mutation, but rather a conversion of an amino acid encoded by the codon comprising the position to be converted with the base editor. Usually, base editors are thus used as molecular complex. Base editors, including, for example, CBEs (base editors mediating C to T conversion) and ABEs (adenine base editors mediating A to G conversion), are powerful tools to introduce direct and programmable mutations without the need for double-stranded cleavage (Komor et al., Nature, 2016, 533(7603), 420-424; Gaudelli et al., Nature, 2017, 551, 464-471). In general, base editors are composed of at least one DNA targeting module and a catalytic domain that deaminates cytidine or adenine. All four transitions of DNA (A→T to G→C and C→G to T→A) are possible as long as the base editors can be guided to the target site. Originally developed for working in mammalian cell systems, both BEs and ABEs have been optimized and applied in plant cell systems. Efficient base editing has been shown in multiple plant species (Zong et al., Nature Biotechnology, vol. 25, no. 5, 2017, 438-440; Yan et al., Molecular Plant, vol. 11, 4, 2018, 631-634; Hua et al., Molecular Plant, vol. 11, 4, 2018, 627-630).

The terms "color tag", "tag", or "label", in the context of the present invention are, used interchangeably herein. A color tag, in this context, is the ability to form and/or exhibit a compound (e.g., any chemical compound from a low molecular weight compound to a high molecular weight protein) that, due to its inherent characteristics, can be used for giving rise to a color signal in a plant or parts of a plant and that allows differentiation of labeled and unlabeled plants, preferably in a certain type of cells, tissues and/or organs and preferably at least in a certain developmental stage. This developmental stage is preferably that early growth stage in which a plant seedling competes for natural resources with weeds in the environment. When the aforementioned compound is used in context of an active color tag, it must be detectably present in the hypocotyl and/cotyledon or otherwise in the canopy of the plant by means as disclosed herein or known to the skilled person. The molecule or compound, formed and/or exhibited as part of the color tag may be a dye or other compound able to confer such a detectable color signal, wherein that compound does not have any significant harmful effects on the plant's growth and development at the used amount and/or concentration and which is preferably environmentally friendly. A color tag, may also give rise to a naturally occurring color of plants, i.e. a genotypically and phenotypically detectable trait, wherein this color trait may preferentially be specifically made available in a plant cell, tissue, organ or whole plant in the developmental stage being the detection stage according to the present invention by means of genome engineering, breeding and the like. Any technique of genome engineering may be used to produce a detectable color signal, for example, by introducing an expressible color trait and/or by modifying a regulatory element, such as a promoter, so that the color tag is active, preferentially specifically active during the detection stage. Generally, the molecule or compound, formed and/or exhibited as part of the color tag, typically comprises a chromophore or fluorophore or a combination thereof. A color tag that uses an external compound as defined above and further explained herein, introduced into the seed of a plant, is referred to as a "chemical color tag". Introduction of the chemical color tag may also be achieved by coating the seed with the respective compound, if said compound can enter the seed and/or plant. A color tag introduced by a genome modification system or by breeding or induction is referred to as a "genomic color tag". Several different color tags may be combined including the combination of chemical color tags and different genomic color tags.

An "active" color tag refers to the state or condition in which the compound formed and/or exhibited as part of the color tag is detectably present in the cotyledon and/or hypocotyl or otherwise in the canopy of the plant, allowing differentiation of the desired plant and undesired weeds. Thus a seed that comprises, e.g. contains or is coated with, a dye, comprises a chemical color tag, which is, however, not active before germination. When the dye is transported e.g. into the hypocotyl or cotyledon in the early germination stage and gives rise to a color signal, the chemical color tag is active. Likewise, a seed comprising a genetic modification, for instance capable of expressing a gene encoding a dye or an enzyme synthetizing a dye, initially comprises an inactive genomic color tag. When the respective gene is indeed expressed and the color signal is present, the genomic color tag is active.

A natural color tag is a color tag based on a dye that is a compound or molecule naturally occurring in a plant species or a dye that is a slightly derivatized form of a compound or molecule that naturally in a plant species.

A "transient color tag" as used herein describes a color tag, wherein the dye is not present, or only in trace amounts, in the harvested product and in the crop plant when it is ready to be harvested if the dye is a compound or molecule naturally occurring in the plant. When the color signal is caused by a dye that naturally exists in the plant, but at a lower concentration, a transient color tag refers to methods, wherein the concentration of the dye in the various parts of the plant is approximately the same or lower than it naturally is in the respective parts of the plant.

A "color signal" in the context of the present invention refers to the presence of a detectable color or coloration including any luminescence signal, comprising a fluorescent, phosphorescence and chemiluminescence signal, conferred by a dye or a dye component or a dye component (donor acceptor) pair used in the context of a color tag. The color signal may comprise a signal in the visible spectrum as well as ultra violet and infra-red light. If the color conferred by the active color tag is similar to the natural coloration of the plant and/or the relevant weed, the quality and/or intensity of the color conferred by the active color tag has to be detectably different (by means as disclosed herein or known to the skilled person) from natural coloration of the plant before introduction or induction of the respective color tag as well as from the relevant weed(s). Preferably, the color conferred by the active color tag is not the natural, inherent main color of the plant before introducing the respective color tag and preferably not the main color of the relevant weed(s).

A "CRISPR nuclease", as used herein, is a specific form of a site-directed nuclease and refers to any nucleic acid guided nuclease which has been identified in a naturally occurring CRISPR system, which has subsequently been isolated from its natural context, and which preferably has been modified or combined into a recombinant construct of interest to be suitable as tool for targeted genome engineering. Any CRISPR nuclease can be used and optionally reprogrammed or additionally mutated to be suitable for the various embodiments according to the present invention as long as the original wild-type CRISPR nuclease provides for DNA recognition, i.e., binding properties. CRISPR nucleases also comprise mutants or catalytically active fragments or fusions of a naturally occurring CRISPR effector sequences, or the respective sequences encoding the same. A CRISPR nuclease may in particular also refer to a CRISPR nickase or even a nuclease-dead variant of a CRISPR polypeptide having endonucleolytic function in its natural environment. A variety of different CRISPR nucleases/systems and variants thereof are meanwhile known to the skilled person and include, inter alia, CRISPR/Cas systems, including CRISPR/Cas9 systems (EP2771468), CRISPR/Cpf1 systems (EP3009511B1), CRISPR/C2C2 systems, CRISPR/CasX systems, CRISPR/CasY systems, CRISPR/Cmr systems, CRISPR/MAD systems, including, for example, CRISPR/MAD7 systems (WO2018236548A1) and CRISPR/MAD2 systems, CRISPR/CasZ systems and/or any combination, variant, or 30 catalytically active fragment thereof. A nuclease may be a DNAse and/or an RNAse, in particular taking into consideration that certain CRISPR effector nucleases have RNA cleavage activity alone, or in addition to the DNA cleavage activity.

A "CRISPR system" is thus to be understood as a combination of a CRISPR nuclease or CRISPR effector, or a nickase or a nuclease-dead variant of said nuclease, or a functional active fragment or variant thereof together with the cognate guide RNA (or pegRNA, crRNA or sgRNA) guiding the relevant CRISPR nuclease.

The "detection stage" as used herein is that developmental stage of a plant, usually during the early growth stage, when a seed or seedling starts to grow out and when the growing seedling then competes with weeds in the surrounding environment for nutrients etc. Particularly during this stage, which may have a varying staring point and length for different crop plants as it is known to the skilled person, a detection is reasonable to be in a position to discriminate a growing plant from a weed by applying cognate detection techniques and to initiate the right measures to control the weeding in a targeted and automatic manner.

The term "dye" in the context of the present invention refers to any compound, molecule or part thereof that gives rise to a detectable color signal. A dye typically comprises a chromophore or fluorophore or a combination thereof. A dye as used herein can be the color tag, or the dye can be comprised by the chemical and/or genomic color tag as part of the chemical and/or genomic color tag.

The term "early growth phase" or "early growth stage" as used herein refer to the time frame of plant growth after onset of germination until, and including, the 4-leaf-stage of the plant. This equals BBCH 14 on the BBCH-scale, which gives the same code for similar growth stages for a range of crop plants (Meier, Uwe: Growth stages of mono- and dicotyledonous plants. BBCH Monograph. Quedlinburg 2018. Open Agrar Repositorium).

The term of "genome engineering" as used herein refers to all strategies and techniques for the genetic modification of any genetic information (DNA and RNA) or genome of a plant cell, comprising genome transformation, genome editing, but also including less site-specific techniques, including TILLING and the like. As such, "genome editing" (GE) more specifically refers to a special technique of genome engineering, wherein a targeted, specific modification of any genetic information or genome of a plant cell. As such, the terms comprise gene editing of regions encoding a gene or protein, but also the editing of regions other than gene encoding regions of a genome. It further comprises the editing or engineering of the nuclear (if present) as well as other genetic information of a plant cell, i.e., of intronic sequences, non-coding RNAs, miRNAs, sequences of regulatory elements like promoter, terminator, transcription activator binding sites, cis or trans acting elements. Furthermore, "genome engineering" also comprises an epigenetic editing or engineering, i.e., the targeted modification of, e.g., DNA methylation or histone modification, such as histone acetylation, histone methylation, histone ubiquitination, histone phosphorylation, histone sumoylation, histone ribosylation or histone citrullination, possibly causing heritable changes in gene expression.

A "genome modification system" as used herein refers to any DNA, RNA and/or amino acid sequence introduced into the cell, on a suitable vector and/or coated on a particles and/or directly introduced, wherein the "genome modification system" causes the modification of the genome of the cell in which it has been introduced. A "genome editing system" more specifically refers to any DNA, RNA and/or amino acid sequence introduced into the cell, on a suitable vector and/or coated on a particles and/or directly introduced, wherein the "genome editing system" comprises at least one component being, encoding, or assisting a site-directed nuclease, nickase or inactivated variant thereof in modifying and/or repairing a genomic target site.

A "genomic target sequence" as used herein refers to any part of the nuclear and/or organellar genome of a plant cell, whether encoding a gene/protein or not, which is the target of a site-directed genome editing or gene editing experiment.

The term "germination" as used herein refers to the sum of events that begin with hydration of the seed and culminate in emergence of the embryonic axis from the seed coat. Thus, a given number of days or weeks "after germination" refers to the specified time after the embryonic axis has emerged from the seed coat.

The term "germplasm" as used herein refers to the genetic material of germ cells of a given organism, species or group of organisms.

The "guide molecule" or "guide nucleic acid sequence" (usually called and abbreviated as guide RNA, crRNA, crRNA+tracrRNA, gRNA, sgRNA, depending on the corresponding CRISPR system representing a prototypic nucleic acid-guided site-directed nuclease system), which recognizes a target sequence to be cut by the nuclease. The at least one "guide nucleic acid sequence" or "guide molecule" comprises a "scaffold region" and a "target region". The "scaffold region" is a sequence, to which the nucleic acid guided nuclease binds to form a targetable nuclease complex. The scaffold region may comprise direct repeats, which are recognized and processed by the nucleic acid guided nuclease to provide mature crRNA. A pegRNAs may comprise a further region within the guide molecule, the so-called "primer-binding site". The "target region" defines the complementarity to the target site, which is intended to be cleaved. A crRNA as used herein may thus be used interchangeably herein with the term guide RNA in case it unifies the effects of meanwhile well-established CRISPR nuclease guide RNA functionalities. Certain CRISPR nucleases, e.g., Cas9, may be used by providing two individual guide nucleic acid sequences in the form of a tracrRNA and a crRNA, which may be provided separately, or linked via covalent or non-covalent bonds/interactions. The guide RNA may also be a pegRNA of a Prime Editing system as further disclosed below. The at least one guide molecule may be provided in the form of one coherent molecule, or the sequence encoding the same, or in the form of two individual molecules, e.g., crRNA and tracr RNA, or the sequences encoding the same.

The term "(micro-)particle bombardment" as used herein, also named "biolistic transfection" or "microparticle-mediated gene transfer" refers to a physical delivery method for transferring a coated microparticle or nanoparticle comprising boost genes, booster polypeptides, genome engineering components, and/or transgenes into a target cell or tissue. The micro- or nanoparticle functions as projectile and is fired on the target structure of interest under high pressure using a suitable device, often called gene-gun. The transformation via particle bombardment uses a microprojectile of metal covered with the construct of interest, which is then shot onto the target cells using an equipment known as "gene gun" (Sandford et al. 1987) at high velocity fast enough (-1500 km/h) to penetrate the cell wall of a target tissue, but not harsh enough to cause cell death. The precipitated construct on the at least one microprojectile is released into the cell after bombardment. The acceleration of microprojectiles is accomplished by a high voltage electrical discharge or compressed gas (helium). Concerning the metal particles used it is mandatory that they are non-toxic, non- reactive, and that they have a lower diameter than the target cell. The most commonly used are gold or tungsten. There is plenty of information publicly available from the manufacturers and providers of gene-guns and associated system concerning their general use.

The term "plant endogenous sequence" refers to a sequence that is endogenous to plant species or a sequence with a certain identity range thereto as long as the sequence still has/encodes the same function as the reference sequence Thus a plant endogenous sequence may be any sequence, that exists in the plant species into/in which a color tag is introduced/induced or exists in a different plant species or a sequence with a certain identity range thereto.

A "plant material" as used herein refers to any material which can be obtained from a plant during any developmental stage. The plant material can be obtained either in planta or from an in vitro culture of the plant or a plant tissue or organ thereof. The term thus comprises plant cells, tissues and organs as well as developed plant. The term also comprises a derivative of the plant material, e.g., a protoplast, derived from at least one plant cell comprised by the plant material. The term therefore also comprises meristematic cells or a meristematic tissue of a plant.

The terms "plant", "plant organ", or "plant cell" as used herein refer to a plant organism, a plant organ, differentiated and undifferentiated plant tissues, plant cells, seeds, and derivatives and progenies thereof. Plant cells include without limitation, for example, cells from seeds, from mature and immature embryos, meristematic tissues, seedlings, callus tissues in different differentiation states, leaves, flowers, roots, shoots, male or female gametophytes, sporophytes, pollen, pollen tubes and microspores, protoplasts, macroalgae and microalgae. Different plant cells, can have any degree of ploidity, i.e. they may either be haploid, diploid, tetraploid, hexaploid or polyploid.

The term "regulatory element" as used herein refers to a nucleic acid sequence that is not part of the protein-encoding nucleotide sequence, but can direct and/or influence the expression of the protein-encoding nucleotide sequence. The term thus refers to core promoter sequence, a proximal promoter sequence, a terminator sequences or polyadenylation signals and the like. Other examples of regulatory sequences are enhancers, silencers, insulators, tethering elements, introns, or cis-regulatory elements, trans regulatory elements or a locus control sequence. A regulatory my also influence splicing of a particular nucleic acid sequence, for instance regulating alternative splicing variants. A regulatory sequence can further be a combination of the above. Depending on the type of regulatory element it is located on the nucleic acid molecule before (i.e., 5' of), after (i.e., 3' of) or between (in case of intronic elements) the protein-encoding nucleotide sequence.

An "RNA-guided nuclease" is a site-specific nuclease, which requires an RNA molecule, i.e. a guide RNA, to recognize and cleave a specific target site, e.g. in genomic DNA or in RNA as target. The RNA-guided nuclease forms a nuclease complex together with the guide RNA and then recognizes and cleaves the target site in a sequence-dependent matter. RNA-guided nucleases can therefore be programmed to target a specific site by the design of the guide RNA sequence. The RNA-guided nucleases may be selected from a CRISPR/Cas system nuclease, including CRISPR/Cpf1 systems, CRISPR/C2C2 systems, CRISPR/CasX systems, CRISPR/CasY systems, CRISPR/Cmr systems, CRISPR/Cms systems, CRISPR/MAD7 systems, CRISPR/MAD2 systems and/or any combination, variant, or catalytically active fragment thereof. Such nucleases may leave blunt or staggered ends. Further included are nickase or nuclease-dead variants of an RNA-guided nuclease, which may be used in combination with a fusion protein, or protein complex, to alter and modify the functionality of such a fusion protein, for example, in a Base Editor or Prime Editor.

The terms "SDN-1", "SDN-2", and "SDN-3" as used herein are abbreviations for the platform technique "site-directed nuclease" 1, 2, or 3, respectively, as caused by any site directed nuclease of interest, including, for example, Meganucleases, Zinc-FingerNucleases (ZFNs), Transcription Activator Like Effector Nucleases (TALENs), and CRISPR nucleases. SDN-1 produces a double-stranded or single-stranded break in the genome of a plant without the addition of foreign DNA. A "site-directed nuclease" is thus able to recognize and cut, optionally assisted by further molecules, a specific sequence in a genome or an isolate genomic sequence of interest. For SDN-2 and SDN-3, an exogenous nucleotide template is provided to the cell during the gene editing. For SDN-2, however, no recombinant foreign DNA is inserted into the genome of a target cell, but the endogenous repair process copies, for example, a mutation as present in the template to induce a (point) mutation. In contrast, SDN-3 mechanism use the introduced template during repair of the DNA break so that genetic material is introduced into the genomic material.

A "site-specific nuclease" herein refers to a nuclease or an active fragment thereof, which is capable to specifically recognize and cleave DNA at a certain location. This location is herein also referred to as a "target sequence". Such nucleases typically produce a double-strand break (DSB), which is then repaired by non-homologous end-joining (NHEJ) or homologous recombination (HR). Site-specific nucleases include meganucleases, homing endonucleases, zinc finger nucleases, transcription activator-like nucleases and CRISPR nucleases, or variants including nickases or nuclease-dead variants thereof.

The terms "transformation", "transfection", "transformed", and "transfected" are used interchangeably herein for any kind of introduction of a material, including a nucleic acid (DNA/RNA), amino acid, chemical, metabolite, nanoparticle, microparticle and the like into at least one cell of interest by any kind of physical (e.g., bombardment), chemical or biological (e.g., Agrobacterium) way of introducing the relevant at least one material.

The term "weeding", in the context of the present invention, refers to the selective removal or extermination of any unwanted plants that grow near or next to desired crop plants. The term "weed" or "weeds" refers to any plant that is considered undesirable in the context of growing plants. Weeds will differ dependent on geographic regions.

### Brief Description of the Figures

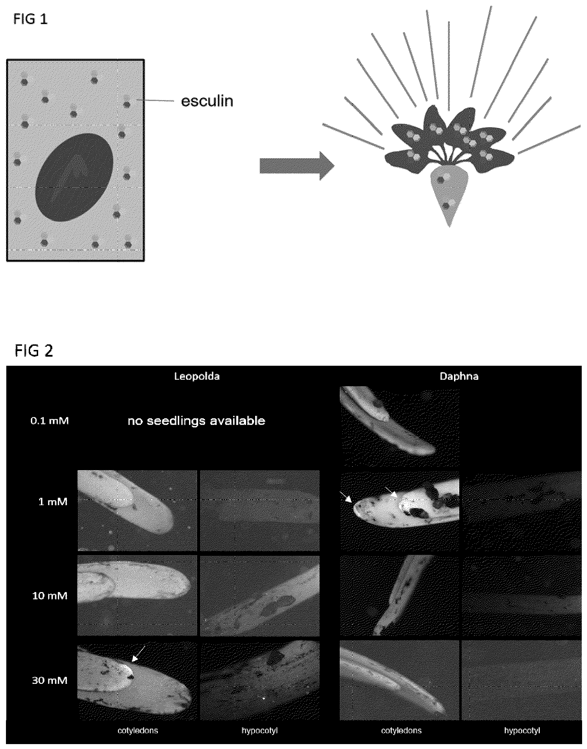

**Figure 1****:** Schematic figure of the incubation of seeds with esculin hydrate resulting in the labeled sugar beet plant which emits blue fluorescence color. Four different concentrations of esculin hydrate (0.1 mM, 1 mM, 10 mM and 30 mM) were used to incubate two varieties of sugar beet seeds (Leopolda and Daphna). Incubation takes place for about 24 hours and plants are checked for blue fluorescence color via fluorescence stereoscopy.
**Figure 2****:** Fluorescence pictures showing Leopolda and Daphna after esculin incubation taken on the day of germination. Four different concentrations of esculin hydrate (0.1 mM, 1 mM, 10 mM and 30 mM) were used to incubate two varieties of sugar beet seeds (Leopolda and Daphna). Left column of each variety is showing the cotyledons and the right column is showing the hypocotyl. The arrows indicate particularly strong color signals.

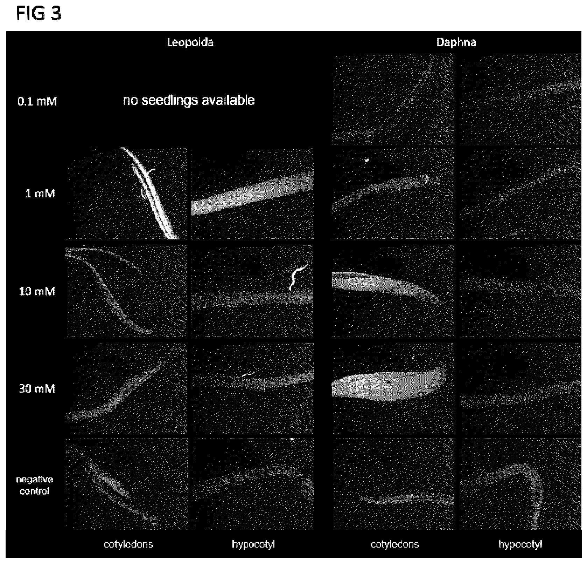

**Figure 3****:** Fluorescence pictures showing Leopolda and Daphna after incubation in esculin hydrate taken three days after germination. Four different concentrations (0.1 mM, 1 mM, 10 mM and 30 mM) were used to incubate two varieties of sugar beet seeds (Leopolda and Daphna). Left column of each type of sugar beet is showing the cotyledons and the right column is showing the hypocotyl.

### Detailed Description

Herein, a method is described to overcome the problem of identifying young seedlings by sensor-based detection independent of leaf shapes or in addition to leaf shape analysis. This is achieved by staining crop plant leaves. The present invention relates to labelling plants using a chemical or genomic color tag, which is already present in the seed and leads to an active color tag in young seedlings. The color tag may be a chemical color tag by introduction of a labelling compound into the seed or a genomic color tag introduced by means a genome modification system or breeding or induced. The detection of the active color tag after germination in the leaves of young seedlings is performed using one or more cognate sensors, such as multispectral of hyperspectral imaging sensors, and may be performed autonomously by weeding platforms. The detected signal can be used to differentiate desired crop plants from unwanted weeds.

In a first aspect the present invention there may be provided a method for labelling a plant, plant cell, tissue, organ, seed, or material with at least one color tag active at least during an early growth stage of a plant and allowing the discrimination of desired plants from undesired weeds by means of color signals, wherein the at least one color tag is detectable by at least one cognate sensor, the method comprising: (i) providing at least one plant cell, or at least one plant, tissue, organ, seed, or material comprising the same; (ii) introducing or inducing at least one color tag into/in the at least one plant cell, or into the at least one plant, tissue, organ, seed, or material comprising the same; (iii) optionally: selecting at least one plant cell, or at least one plant, tissue, organ, seed, or material comprising the same, wherein the selection is based on the successful integration of the color tag; (iv) obtaining at least one plant cell, or at least one plant, tissue, organ, seed, or material comprising the same, that comprises the color tag, so that the color tag is active during the early growth stage of a plant; and (v) optionally: seeding the plant seed and discriminating the desired plants comprising an active color tag from undesired weeds; wherein the color tag is introduced in a transient way, or wherein the color tag is a natural color tag , or any combination thereof.

For a method according to the present invention, the color tag does not necessarily have to be active throughout the entire early growth phase and/or later phases. It is sufficient that the color tag is active in an early growth stage at some point in time that allows the detection of the active color tag during a detection stage, i.e. a sufficient part of the plant exhibiting the color signal is above ground so that is can be detected by a sensor recognizing the cognate signal of or associated with the color tag.

A successful integration of a color tag in a plant, plant cell, tissue organ seed or material does not necessarily complete, i.e. the colortag only has to be integrated into the necessary parts, such as the seed coat or a meristematic cell as further discussed below. When the method is used for a plurality of plants, plant cells, tissues, organs, seeds or materials the selection step may constitute a partial or coarse selection, wherein the integration rate in that plurality of plants, plant cells, tissues, organs, seeds or materials is less than 100 %, for example in a range from about 70% to about 99.99%, from about 75% to about 99.99%, from about 80% to about 99.99%, from about 80% to about 99.99%, from about 85% to about 99.99%, from about 90% to about 99.99%, from about 95% to about 99.99%, or from more than 96%.

In a preferred embodiment of the first aspect of the present invention, the color tag is introduced by labelling a plant seed with a chemical color tag, or by introducing a genomic color tag, or any combination thereof.

In preferred embodiments of the first aspect of the present invention the early growth stage is the time period up to the 4-leaf-stage.

In one embodiment of the first aspect of to the present invention, the color tag is a chemical color tag, preferably wherein the chemical color tag consists of or comprises a dye being selected from the group of a coumarin, including scopoletin or esculetin, more preferably wherein the chemical color tag consists of or comprises a dye being coupled to glucose, including esculin or scopolin.

In embodiments that use a chemical color tag, a fertilized ovule, preferably a seed, is treated with a labelling compound according to the present invention. The fertilized ovule or seed can be incubated in a liquid staining solution containing comprising the labelling compound as shown in the examples. The fertilized ovule, preferably the seed, may also be coated with the labelling compound. It is necessary that the seed and/or fertilized ovule and/or the growing plant can take up the labelling compound. Based on the herein presented data, esculin hydrate but also many other compounds can be used for the accurate staining of crop plants. It has been shown in experiments that esculin hydrate is able to pass the testa of the seeds and enters the embryo. Moreover, esculin hydrate is kept in the tissues of the seedling after germination and is detectable under a fluorescence stereoscope. In conclusion, the fact that a fluorescence molecule can be brought into the plant is of high relevance and can change many technical fields in in agricultural business in the future.

In certain embodiments, the labelling compound is a glucose-coupled dye. Glucose mediated uptake is shown for Esculin in the examples below, but is also described for Fraxin, 6-Carboxyfluorescin-glucose or Glc-1,4,7,10-tetraazacyclododecane-1,4,7,10-tetraaceticacid-(DOTA)-Dy(III). The coupling to glucose is one method that allows the uptake and transportation within the cell. Alternatively, in certain embodiments the dye may enter the seed and/or fertilized ovule and/or the growing plant by different means, such as by virtue of its membrane permeability due physico-chemical properties, such as hydrophobicity. Such labelling compounds may for instance be Scopoletin, MDY-64, Pontamine, Fast Scarlet 4B, FM4-64 (N-(3-Triethylammoniumpropyl)-4-(6-(4-(Diethylamino) Phenyl) Hexatrienyl), neutral red (3-Amino-7-dimethylamino-2-methylphenazin·Hydrochlorid), Neonicotinoids.

In certain embodiments, the dye may not enter the seed and/or fertilized ovule directly, but may be taken up by the growing plant after germination. In such an embodiment the seed may be coated, wherein the coating comprises at least one dye, which is later taken up by the growing plant after germination, for example by release into the soil from the coating and subsequent uptake via the roots of the growing plant. The person skilled in the art knows about various techniques of coating seeds of monocotyledonous and dicotyledonous plants with at least one layer, wherein the dye can be comprised by any one of the layers of the coating (in case more than one layer is used).

In certain embodiments of the present invention the at least one dye is produced by seed-associated microbes and/or endophytes. In these embodiments the seed may be coated, wherein the coating comprises at least one a microbe and/or endophyte producing and optionally releasing the at least one dye.

In embodiments in which the seed or seeds is/are coated, additional adjuvants can be included in the seed coating, e.g. for improved uptake of the at least one dye by the growing plant.

An underlying problem in embodiments, wherein a chemical color tag is used, is that the used dye should preferably not be present in the harvested plant or its harvested product, because remaining dye might impair the quality of the final product. This is especially true if the plant is used for food production and potential toxicity after consumption may be an issue. Therefore, it is imperative in this context of the present disclosure that any dyes, for which safety of other quality concerns of the harvested product might be an issue, is only present in the early growth phase. As a chemical color tag is only added to the seed once, the used dye can be present in a concentration sufficient to give rise to a detectable color signal in the early growth phase while it is only present in trace amounts, if at all, in the grown plant and/or the harvested product. Thus, a chemical color tag is preferentially a transient color tag.

In a further embodiment of the first aspect according to the present invention the color tag is a genomic color tag introduced by a genome modification system by (a) introducing at least one insertion nucleotide sequence, wherein the expression of said insertion nucleotide sequence causes an altered level of at least one compound detectable by at least one cognate sensor, wherein the at least one insertion nucleotide sequence is a plant endogenous sequence, or by (b) modifying or introducing at least one regulatory region that causes an altered expression of an endogenous nucleotide sequence, wherein the altered expression of said endogenous nucleotide sequence causes an altered level of at least one compound detectable by at least one cognate sensor, or any combination of (a) and (b). Preferably a genomic color tag is a transient color tag.

In one embodiments of the present invention, the at least one insertion nucleotide sequence encodes at least one dye.

In another embodiments of the present invention, the at least one insertion nucleotide sequence encodes at least one enzyme, or a cofactor of an enzyme or a different factor that influences the activity of an enzyme, that is involved in the synthesis pathway of the dye or that otherwise catalyzes or facilitates the conversion or modification of a substance resulting in such a dye. In preferred embodiments, the insertion nucleotide gives rise to a transient color tag. In certain embodiments two or more insertion nucleotide sequence encodes encoding two or more different enzymes and/or polypeptides and/or a cofactors of an enzyme and/or different factors that influences the activity of an enzyme in the synthesis pathway of at least one dye are introduced to give rise to a, preferably transient, color tag.

In all embodiments using an insertion nucleotide sequence, wherein the expression of said insertion nucleotide sequence causes an altered level of at least one dye, the insertion is performed in a manner that allows functional expression. The inserted sequence may comprise all regulatory sequences necessary for efficient transcription and optionally translation operably linked to the nucleotide sequence to be expressed. Alternatively the nucleotide sequence to be expressed may be inserted without all regulatory sequences necessary for efficient transcription and optionally translation if the insertion lead to an operably linked position relative to regulatory sequences present in the target genome and thereby the efficient transcription and optionally translation are achieved. The skilled person is well aware of suitable regulatory sequences.

In embodiments using a modification or introduction of at least one regulatory sequence, the introduced or modified regulatory sequence preferentially leads to an increase of the level and/or concentration of a dye. It may however also be envisaged that the genomic color tag is the reduction of a naturally occurring compound, for instance a pigment, that affects the coloration of a plant. Preferentially at least one regulatory element, such as a promoter, enhancer or silencer is modified, inserted and/or exchanged to cause an increased or decreased expression of at least one nucleotide sequence encoding at least one dye or at least one enzyme or factor that is involved in the synthesis pathway of the dye or that otherwise catalyzes or facilitates the conversion or modification of a substance resulting in such a dye.

In some embodiments of the present invention, the insertion of at least one regulatory sequence, possibly by exchanging an existing regulatory sequence or a part thereof, may be performed by using a repair template as described herein or known to the skilled person. In preferred embodiments, the existing promoter sequence(s) of at least one enzyme or polypeptide or cofactor of an enzyme or different factor that influences the activity of an enzyme that is involved in the synthesis pathway of the dye or that otherwise catalyzes or facilitates the conversion or modification of a substance resulting in such a dye is exchanged with a natural or artificial promoter sequence leading to an increased expression of at least one nucleotide sequence encoding said at least one enzyme, polypeptide, cofactor or influencing factor. In some embodiments of the present invention, modifying a regulatory sequence may be achieved by changing or deleting the nucleic acid sequence, or parts thereof, of the regulatory sequence, including the complete deletion of a regulatory sequence, for instance a silencer. In some embodiments of the present invention, modifying a regulatory sequence is achieved by inserting nucleic acid sequences into or next to or near the regulatory sequence, thereby altering the effect or degree of effect of the regulatory sequence.

In one embodiments of the present invention, at least one regulatory sequence is modified by altering the level of at least effector molecule, such as a transcription factor or transcription activator or transcription inhibitor, that binds to the at least one regulatory sequence wherein the at least one effector molecule causes an altered expression of at least one nucleotide sequence encoding at least one dye or at least one enzyme or polypeptide or cofactor of an enzyme or different factor that influences the activity of an enzyme that is involved in the synthesis pathway of the dye or that otherwise catalyzes or facilitates the conversion or modification of a substance resulting in such a dye. In some embodiments of the present invention, a nucleic acid sequence encoding a natural or synthetic effector molecule, may be introduced by using a repair template as described herein or known to the skilled person, wherein the effector molecule in turn increases or decreases the expression rate of least one nucleotide sequence encoding at least one dye or at least one enzyme or polypeptide or cofactor of an enzyme or different factor that influences the activity of an enzyme that is involved in the synthesis pathway of the dye or that otherwise catalyzes or facilitates the conversion or modification of a substance resulting in such a dye. A nucleotide sequence encoding the at least one effector molecule is an endogenous, exogenous or artificial sequence. In certain embodiments of the present invention a regulatory sequence, or parts thereof, is inserted, exchanged, modified or deleted, wherein the insertion, exchange, modification or deletion causes an increased or decreased expression of at least one nucleotide sequence encoding at least one effector molecule, such as a transcription factor or transcription activator or transcription inhibitor, that binds to the at least one regulatory sequence, wherein the at least one effector molecule causes an increased or decreased expression of at least one nucleotide sequence encoding at least one dye or at least one enzyme or polypeptide or cofactor of an enzyme or different factor that influences the activity of an enzyme that is involved in the synthesis pathway of the dye or that otherwise catalyzes or facilitates the conversion or modification of a substance resulting in such a dye. Thus, the transcription and/or translation rates for such an effector molecule may be increased or decreased by inserting or modifying at least one regulatory sequence regulating the expression of a nucleotide sequence encoding said effector molecule, which in turn increases or decreases the expression rate of the at least one nucleotide sequence encoding said at least one dye or enzyme, polypeptide, cofactor or influencing factor.

In certain embodiments, the expression of two or more nucleotide sequence encoding two or more enzymes and/or polypeptides and/or cofactors of an enzyme and/or different factor that influences the activity of an enzyme of the synthesis pathway of at least one dye is increased by modification or introduction of a regulatory sequence.

In certain embodiments, different methods to insert or modify at least one regulatory sequence are combined.

In certain embodiments of the present invention, modification or introduction of a regulatory sequence causes a reduced expression of at least one nucleotide sequence encoding at least one enzyme an or polypeptide that converts at least one dye into a different molecule or compound, thereby increasing the level of the at least one dye (for instance an intermediate in a synthesis pathway) by decreasing the level of said at least enzyme an or polypeptide. In preferred embodiments the reduction of the level of least enzyme or polypeptide that converts at least one dye into a different molecule or compound is combined with: (a) Introducing at least one insertion nucleotide that encodes the at least one dye or at least one different enzyme or polypeptide or cofactor of an enzyme or different factor that influences the activity of an enzyme that is involved in the synthesis pathway of the at least one dye or that otherwise catalyzes or facilitates the conversion or modification of a substance resulting in such a dye; or (b) modification or introduction of a regulatory sequence causing an increased expression of at least one nucleotide sequence encoding at least one different enzyme or polypeptide or cofactor of an enzyme or different factor that influences the activity of an enzyme that is involved in the synthesis pathway of the at least one dye or that otherwise catalyzes or facilitates the conversion or modification of a substance resulting in such a dye. Thereby, for instance, the expression of one or more nucleotide sequences encoding one or more enzymes or polypeptides in a synthesis pathway may be increased leading to increased synthesis of at least one dye (e.g. an intermediate in said synthesis pathway) while at the same time the expression of at least one nucleotide sequence encoding at least one enzyme or polypeptide that converts at least one dye into a different molecule or compound is decreased.

Modifying a regulatory sequence typically leads to an increased or decreased binding affinity of an effector molecule but my also lead to an altered three-dimensional DNA structure e.g. looping and the like, which allows access and thus modulation of pathways or genes involved in the color tag production.

Regulatory sequences that may be introduced as described above to give rise to a color signal may for example comprise a ZmUbi1 promoter, a BdUbi10 promoter, a ZmEf1 promoter, a double 35S promoter, a rice U6 (OsU6) promoter, a rice actin promoter, a maize U6 promoter, a PcUbi4 promoter, a Nos promoter, an AtUbi10 promotor, a BdEF1 promoter, an MeEF1 promoter, an HSP70 promoter, an EsEF1 promoter, an MdHMGR1 promoter or a combination thereof.

In certain embodiments, the at least one introduced regulatory sequence is a tissue-specific promoter, preferentially a leaf-tissue-specific promoter. A tissue-specific promoter, as it is known to the skilled person, causes expression of an operably linked nucleic acid sequence, wherein the expression is either specific to at least one tissue or part of the plant or elevated in at least one tissue or part of the plant compared to the average expression rate in the plant.

In certain embodiments, the at least one introduced regulatory sequence is a developmental-phase-specific promoter. A developmental-phase-specific promoter, as it is known to the skilled person, causes expression of an operably linked nucleic acid sequence, wherein the expression is either specific to at least one developmental phase or elevated in at least one developmental phase compared to the average expression rate in the plant. A developmental-phase-specific promoter, used according to the present invention, causes specific or elevated expression at least during the early growth phase or at least during a part thereof.

In certain embodiments of the first aspect of the present invention the genome modification system comprises at least one site-directed nuclease, nickase or an inactivated nuclease, preferably a nucleic acid guided nuclease, nickase or an inactivated nuclease, or a sequence encoding the same, and optionally at least one guide molecule, or a sequence encoding the same.

A variety of suitable genome editing systems that can be employed according to the methods of the present invention is available to the skilled person and can be easily adapted for use in the methods used herein. The skilled person may for instance use Cas9-based system as described in EP2771468 or a Cpf1-based system as described in EP3009511 or a system using base editors as described in Rees et al., Base editing: precision chemistry on the genome and transcriptome of living cells, Nat Rev Genet. 2018 Dec; 19(12): 770-788.

In certain embodiments using a genomic color tag, the modified cell according to the methods of the present invention is a haploid cell, wherein the haploid cell is generated by introducing a genome editing system into at least one cell, preferably an IIM cell, to be modified, wherein the genome editing system is capable of introducing at least one mutation into the genomic target sequence of interest resulting in a cell having haploid inducer activity. Various methods for doubling chromosomes in plant biotechnology are available to the skilled person for various cultivars. In one embodiment, chromosome doubling can be achieved by using colchicine treatment. Other chemicals for chromosome doubling, are available for use according to the methods disclosed herein, wherein these chemicals may be selected from antimicrotubule herbicides, including amiprophosmethyl (APM), pronamide, oryzalin, and trifluralin, which are all known for their chromosome doubling activity.

In certain embodiments, there is provided a method comprising a regeneration step, wherein the regeneration may be performed either by direct meristem organogenesis, i.e., by directly obtaining a viable plant cell, tissue, organ, plant or seed modified as detailed above, or wherein the regeneration may be performed indirectly, i.e., via an additional cell culture step proceeding through callus organogenesis.

In certain embodiments, where at least one genome editing system is introduced according to the methods disclosed herein, the methods include the introduction of at least one site-directed nuclease, nickase or an inactivated nuclease, or a sequence encoding the same, wherein the site-directed nuclease, nickase or an inactivated nuclease may be selected from the group consisting of a CRISPR nuclease or a CRISPR system, including a CRISPR/Cas system, preferably from a CRISPR/MAD7 system, a CRISPR/Cfp1 system, a CRISPR/MAD2 system, a CRISPR/Cas9 system, a CRISPR/CasX system, a CRISPR/CasY system, a CRISPR/Cas13 system, or a CRISPR/Csm system, a zinc finger nuclease system, a transcription activator-like nuclease system, or a meganuclease system, or any combination, variant, or catalytically active fragment thereof.

In certain embodiments In certain embodiments, where at least one genome editing system is introduced according to the methods disclosed herein, the method additionally comprises the introduction of regeneration booster. A variety of regeneration boosters suitable for application in the methods disclosed herein are known to the skilled person.

In certain embodiments, wherein at least one genome editing system is introduced, the at least one genome editing system may further comprise at least one reverse transcriptase and/or at least one cytidine or adenine deaminase, preferably wherein the at least one 5 cytidine or adenine deaminase is independently selected from an apolipoprotein B mRNA-editing complex (APOBEC) family deaminase, preferably a rat-derived APOBEC, an activation-induced cytidine deaminase (AID), an ACF1/ASE deaminase, an ADAT family deaminase, an ADAR2 deaminase, or a PmCDA1 deaminase, a TadA derived deaminase, and/or a transposon, or a sequence encoding the aforementioned at least one enzyme, or 10 any combination, variant, or catalytically active fragment thereof.

In embodiments, wherein the site-directed nuclease or variant thereof is a nucleic acid-guided site-directed nuclease, the at least one genome editing system additionally includes at least one guide molecule, or a sequence encoding the same.

In certain embodiments, the genome editing system may be a base editor (BE) system.

In other embodiments, the genome editing system may be a Prime Editing system.

Any nucleic acid sequence comprised by, or encoding a genome modification or genome editing system disclosed herein, may be "codon optimized" for the codon usage of a plant target cell of interest. This means that the sequence is adapted to the preferred codon usage in the organism that it is to be expressed in, i.e. a "target cell of interest", e.g. an IIM cell, which may have its origin in different target plants (wheat, maize, sunflower or sugar beet for example) so that a different codon optimization may be preferable, even though the encoded effector on protein level may be the same. If a nucleic acid sequence is expressed in a heterologous system, codon optimization increases the translation efficiency significantly.

In a further embodiment using a genome editing system according to any of the embodiments described above, the system may additionally comprise at least one repair template, or a sequence encoding the same. A "repair template", "repair nucleic acid molecule", or "donor (template)" refers to a template exogenously provided to guide the cellular repair process so that the results of the repair are error-free and predictable. In the absence of a template sequence for assisting a targeted homologous recombination mechanism (HDR), the cell typically repairs a genomic DNA break via the error-prone process of non-homologous end-joining (NHEJ).

In one embodiment, the at least one repair template may comprise or encode a double- and/or single-stranded sequence.

In another embodiment, the at least one repair template may comprise symmetric or asymmetric homology arms.

In a further embodiment, the at least one repair template may comprise at least one chemically modified base and/or backbone, including a phosphothioate modified backbone, or a fluorescent marker attached to a nucleic acid of the repair template and the like.

In one embodiment, a genome modification or editing system according to any of the embodiments described above, the at least one site-directed nuclease, nickase or an inactivated nuclease, or a sequence encoding the same, and/or optionally the at least one guide nucleic acid, or the sequence encoding the same, and/or optionally the at least one repair template, or the sequence encoding the same, are provided simultaneously, or one after another.

At least one repair template can be delivered with the at least one genome modification or editing system simultaneously or subsequently with the proviso that it will be active, i.e., present and readily available at the site of a genomic target sequence in an IIM cell to be modified together with the at least one further tools of interest.

The repair template can be additionally introduced by bombardment at least one more times 1-8 hours after first bombardment, especially when genome editing components are delivered as sequences encoding the same to increase repair template availability for a targeted repair process.

In certain embodiments, the at least one genome editing system and optionally the at least one repair template, or the respective sequences encoding the same, are introduced transiently or stably, or as a combination thereof. While a stable integration of at least one genome editing system, in particular the site-directed nuclease or variant thereof, but not necessarily including at least one guide RNA, may allow a stable expression of this effector, the methods as disclosed herein can be performed in a full transient way. This implies that the tools as such are not integrated into the genome of a cell to be modified, unless at least one repair template is used. This transient approach may be preferably for a highly controllable gene editing event.

Plant cells for use according to the methods disclosed herein can be part of, or can be derived or isolated from any type of plant inflorescent meristems in intro, or in vivo. It is possible to use isolated plant cells as well as plant material, i.e. whole plants or parts of plants containing the plant cells. A part or parts of plants may be attached to or separated from a whole intact plant.

In certain embodiments, plant growth regulators like auxins or cytokinines in the tissue culture medium can be added to manipulate and/or to induce callus formation and subsequently changed to induce embryos to form from the callus.

Somatic embryogenesis has been described to occur in two ways: directly or indirectly. Direct embryogenesis occurs when embryos are started directly from explant tissue creating an identical clone. Indirect embryogenesis occurs when explants produced undifferentiated, or partially differentiated, cells (i.e. callus) which then is maintained or differentiated into plant tissues such as leaf, stem, or roots.

A variety of delivery techniques may be suitable according to the methods of the present invention for introducing the components of a genome modification or editing system and/or at least one transgene, or the respective sequences encoding the same, into a cell, in particular an IIM cell, the delivery methods being known to the skilled person, e.g. by choosing direct delivery techniques ranging from polyethylene glycol (PEG) treatment of protoplasts, procedures like electroporation, microinjection, silicon carbide fiber whisker technology, viral vector mediated approaches and particle bombardment. A common biological means, and a preferred cargo according to the present invention, is transformation with Agrobacterium spp. which has been used for decades for a variety of different plant materials. Viral vector mediated plant transformation represents a further strategy for introducing genetic material into a cell of interest.

A particularly preferred delivery technique may be the introduction by physical delivery methods, such as (micro-)particle bombardment or microinjection. Particle bombardment includes biolistic transfection or microparticle-mediated gene transfer, which refers to a physical delivery method for transferring a coated microparticle or nanoparticle comprising a nucleic acid or a genetic construct of interest into a target cell or tissue. Means of physical introduction are suitable to introduce nucleic acids, i.e., RNA and/or DNA, and proteins. Particle bombardment and microinjection have evolved as prominent techniques for introducing genetic material into a plant cell or tissue of interest. Helenius et al., "Gene delivery into intact plants using the Helios™ Gene Gun", Plant Molecular Biology Reporter, 2000, 18 (3):287-288 discloses a particle bombardment as physical method for introducing material into a plant cell.

In one embodiment using particle bombardment, the various components of a genome modification or editing system and/or at least one booster gene and/or at least one transgene, or the respective sequences encoding the same, are co-delivered via microcarriers comprising gold particles having a size in a range of 0.4-1.6 micron (pm), preferably 0.4-1.0 pm. In an exemplary process, 10-1,000 pg of gold particles, preferably 50-300 pg, are used per one bombardment.

The various components of a genome modification or editing system or the respective sequences encoding the same, can be delivered into target cells for example using a Bio-Rad PDS-1000/He particle gun or handheld Helios gene gun system. When a PDS-1000/He particle gun system used, the bombardment rupture pressures are from about 450 psi to 2200 psi, preferred from about 450 to 1800 psi, while the rupture pressures are from about 100 to 600 psi for a Helios gene gun system. More than one chemical or construct can be co-delivered with genome engineering components into target cells simultaneously. The above-described delivery methods for transformation and transfection can be applied to introduce the tools of the present invention simultaneously. Likewise, specific transformation or transfection methods exist for specifically introducing a nucleic acid or an amino acid construct of interest into a plant cell, including electroporation, microinjection, nanoparticles, and cell-penetrating peptides (CPPs). Furthermore, chemical-based transfection methods exist to introduce genetic constructs and/or nucleic acids and/or proteins, comprising inter alia transfection with calcium phosphate, transfection using liposomes, e.g., cationic liposomes, or transfection with cationic polymers, including DEAD-dextran or polyethylenimine, or combinations thereof. The above delivery techniques, alone or in combination, can be used for in vivo (including in planta) or in vitro approaches. Particle bombardment may have the advantage that this form of physical introduction can be precisely timed so that the material inserted can reach a target compartment together with other effectors in a concerted manner for maximum activity. IIM cells were shown to be particularly susceptible to particle bombardment and tolerate this kind of introduction well.

In agrobacterial transformation, the Agrobacteria first find the suitable cells and attach to the plant cell walls, which is generally referred as "inoculation". Following the inoculation, the Agrobacteria are growing with plant cells under suitable conditions for a period of time - from several hours to several days - to allow T-DNA transfer. Agrobacterium-plant interaction, plant tissue structure, plant cell type, etc. constrain agrobacterial transformation. Limited by plant cell susceptibility and accessibility it is generally believed that Agrobacterium-mediated transformation is plant species, plant tissue-type and plant cell-type dependent. Conversely, based on physical forces particle bombardment is -at least in theory - plant species and plant cell-type independent, and is able to transform any cells when appropriate pressure applied.

Plant stem cells are mainly located on a specialized tissue named plant meristem, including shoot, root meristem, and inflorescence meristem.

For important cereal crops (e.g., maize, wheat, rye, oat, barley, sorghum, rice), the most widely used explant for genome engineering is immature zygotic embryo.

In one embodiment, more than one different transformation/transfection technique as disclosed above are combined, preferably, wherein at least one of the components of a genome modification or editing system and/or at least one booster gene and/or at least one transgene, or the respective sequences encoding the same, is introduced via particle bombardment.

In certain embodiments, the methods for plant genome modification as disclosed herein may comprise a preceding step of preparing plant cells as part of preferably immature inflorescence meristem (IIM) for providing at least one immature inflorescence meristem cell.

In certain embodiments of the methods disclosed herein, the regeneration of the at least one modified cell may be a direct meristem regeneration comprising the steps of: shoot meristem induction for about 1 to 4 weeks, preferably 10 to 25 days, shoot meristem propagation for about 1 to 4 weeks, preferably 10 to 25 days, shoot outgrowth for about 1 to 4 weeks, preferably 10 to 20 days, and root outgrowth for about 1 to 4 weeks, preferably 3 to 20 days.

In other embodiments of the methods disclosed herein, the regeneration of the at least one modified cell may be an indirect meristem regeneration comprising the steps of: inducing embryogenic callus formation for about 1 to 6 weeks, preferably 2 to 4 weeks, most preferably 3 weeks, shoot meristem development and outgrowth for about 1 to 6 weeks, preferably 2 to 4 weeks, most preferably 10 to 25 days, and root outgrowth for about 1 to 4 weeks, preferably 3 to 14 days; and optionally: screening for genetic modification events in the regenerated T0 plants; and further optionally: growing the modified T0 plants for T1 seed production and optionally screening T1 progeny for desirable genetic modification events.

In certain embodiments, the introduction may preferably be into at least one plant immature inflorescence meristem (IIM) cell and may be mediated by biolistic bombardment.

Preferably, all exogenously provided elements ortools of a genome or gene editing system, or sequences encoding the same, and optionally provided repair template sequences are provided either simultaneously or subsequently, wherein the terms simultaneously and subsequently refers to the temporal order of introducing the relevant at least one tool, which may be introduced to be expressed transiently or in a stable manner, with the proviso that both simultaneous and subsequent introduction guarantee that one and the same IIM cell will comprise the relevant tools in an active and/or expressible manner. Ultimately, all genome modification or gene editing system elements are thus physically present in one IIM cell.

In certain embodiments, a color tag can be introduced into a germplasm of interest by classical breeding strategies. In turn, the color tag will be available in the resulting plant, plat cell, tissue, organ or seed used according to the methods of the present invention.

In certain embodiments of the first aspect according to the present invention, the at least one cognate sensor is an image sensor, optionally included in a camera, wherein the image sensor is an RGB sensor, a multispectral sensor, a hyperspectral sensor, a fluorescence sensor, or a monochrome sensor, or a combination of at least two sensors, wherein the at least one cognate sensor may optionally comprise an irradiation unit for irradiating at least one plant, plant cell, tissue, organ, seed, or material with light, wherein said modulated irradiation source is preferably used in combination with the image sensor.

The image sensor may comprise a color sensor. A color sensor can be used for the selective detection and evaluation of the visible and optionally the ultra violet and infrared spectral range. A color sensor may for instance be provided in the form of a LAB color sensor, an RGB (red-green-blue) sensor or a true color sensor. The principle of an image of the color sensor may be based on a color filter array or a color mosaic array for capturing color information. The color filter filters the light by wavelength range such that separate filtered intensities include information about the color of light. An RGB sensor - also called Bayer filter - is an example of above-mentioned color filters and gives information about the intensity of light in red, green and blue wavelength regions. A raw image data captured by said RGB Sensor can be converted to a full-color image by a demosaicing algorithm. Further examples for above mentioned color filters are RGBE filter, CYYM filter, CYGM filter, RGBW Bayer filter, RGBW#1 filter, RGBW#2 filter, RGBW#3 filter or X-Trans filter.

Preferentially, the image sensor comprises a multi- or hyperspectral imaging sensor that collects and processes information from across the electromagnetic spectrum for obtaining the spectrum for each pixel in an image of the field, with the purpose of identifying and detecting desired plants and distinguishing them from weeds. In particular, the image sensor may be integrated into a push broom scanner. It is particularly preferred that the captured image data are high-resolution. Different types of sensors and/or cameras, e.g. an RGB sensor and a hyperspectral imaging sensor may be used in combination.

Multiple recording can describe recording one individual picture element at least double or triple or quadruple or multiple from one perspective in different color and/or wavelength ranges. Alternatively, multiple recording can preferably describe recording one individual picture element at least double or triple or quadruple or multiple from different perspectives. It is preferred that the multiple recording can be made while moving the hyperspectral image sensor and/or the image sensor.

The detection of a color signal may be preceded by an optional step of irradiation the plant or relevant part of the plant, such as the hypocothyl and/or cotyledon or otherwise the canopy of the plant. This irradiation step may simple use white light with various combination of wavelength or ranges of wavelengths or may use a particular wavelength or range of wavelengths or sets of different wavelengths or different ranges of wavelength. This irradiation step can serve to reduce sunlight effects by providing similar illumination the analyzed plant or part of the plant even under different sunlight conditions. When a fluorescent compound is used chemical color tag the light used for irradiation needs to be suitable for excitation of the fluorophore. Excitation and Emission spectra of a plethora of conventionally used fluorophore compounds is known to the skilled person. Typically, the excitation of a fluorophore and the subsequent detection of the emitted fluorescent light uses a set of optical filters. Optical filters or sets of optical filters together with the relevant wavelength information (bandpass or cut-off) are well known in the field and easily available to the skilled person. The light for exciting the fluorophore should contain a wavelength or range of wavelengths around the absorbance peak of the fluorophore (e.g. achieved by a bandpass excitation filter). The wavelength or range of wavelengths used for exciting the fluorophore should not, or only minimally, comprise a wavelength or range of wavelength that is used for detecting the fluorophore, typically around the emission peak of the used fluorophore. When an irradiation step is performed, the sensor detecting the color signal may have an emission filter (using a wavelength cut-of or bandpass) for filtering out wavelengths other than the wavelength or range of wavelength used to detect the color tag.

In certain embodimens of the first aspect according to the present invention, the at least one undesired weed is selected from the group consisting of *Alopecures myosuroides, Acalypha australis L., Amaranthus retroflexus, Agropyron repens (L) Beauv., Agrostis gigantea Roth. Fl. Germ, Brassica napus, Amaranthus blitoide, Amaranthus lividus Loisel., Amaranthus palmerii, Amaranthus patulus bertoloni, Amaranthus powellii, Capsella bursa-pastoris, Ambrosia artemisiifolia, Ambrosia trifida, Amethystea caerulea L., Anthemis arvensis L., Anthemis cotula L., Artemisia scoparia, Artemisia sieversiana, Chenopodium album, Fumaria officinalis, Galeopsis tetrahit, Barbarea vulgaris R.Br., Bidens tripartite, Galium aparine, Calystegia hederacea Wall, Calystegia sepium, Geranium spp., Cenchrus echinatus, Centaurea cyanus, Lamium purpureum, Chenopodium glaucum L., Chenopodium serotinum L., Matricaria camomilla, Cirsuim vulgare, Commelina communis L., Conium maculatum, Matricaria inodora, Cuscuta campestris (pentago-na), Cuscuta pentagona Engelm., Cyperus esculentus, Datura stramonium L., Digitaria sanguinalis, Digitaria violascens Link, Mercurialis annual, Echinochloa phyllopogon Stapf, Eleusine inica(L) Gaertn., Elsholtzia ciliata (Thunb.) Hylander, Poa Annua, Polygonum aviculare, Erigeron annuus L., Erigeron canadensis L., Erysimum cheiranthoides L., Fallopia japonica, Polygonum convolvu-lus, Polygonum persicaria, Galinsoga parviflora, Senecio vulgaris, Solanum nigrum, Helianthus annuus, Hibiscus trionum, Hordeum vulgare, Iva annua, Kochia scoparia, Stellaria media, Thlaspi arvense, Veronica arvensis, Viola arvensis, Abutilon theophrasti, Aethusa cynapium, Myosoton aquaticum (L.) Moench, Oxalis corniculata L., Panicum capillare, Panicum dichotomiflorum, Persicaria convolvulus L., Persicaria lapathifolia (L.) S.F.Gray, Persicaria longiseta (De Bruyn) Kitag., Persicaria nepalensis (Meisn.) H.Gross, Persicaria vulgaris Webb et Moq., Phragmites australis (Cav.) Trin. Ex Steud., Atriplex patula, Cirsium arvense (Cirsium Segetum* / *Cephalonoplos segetum), Echinochloa crus-galii, Polygonum bungeanum T., Elymus repens* / *Elytrigia repens, Polygonum lapatifolium, Polygonum pensylvaticum, Myosotis arvensis, Portulaca oleracea, Rhaponticum repens, Rorippa atrovirens Ohwi et Hara, Rorippa islandica (Oeder) Borlas, Rorippa sylvestris Besser, Rumex acetosella L., Rumex crispus L., Rumex japonicus Houtt., Rumex longifolius DC., Rumex obtusifolius L., Sagina japonica (Sw.) Ohwi, Salsola tragus, Salvia reflexa, Sonchus arvensis (brachyotus DC), Setaria glauca, Setaria viridis, Atriplex hastate, Avena fatua, Solanum ptycanthum, Solanum rostratum, Solanum sarrachoides, Convolvulus arvensis, Equisetum arvense, Sonchus brachyotis DC., Spergula arvensis L., Malva spp., Stellaria alsine Grimm var.undulata (Thunb.) Ohwi, Polygonum amphibium, Symphytum officinale L., Taraxacum (officinale and mongolicum), Sinapis arvensis, Solanum tuberosum, Stachys arvensis, Veronica hederifolia L., Veronica persica, Urtica urens, Xanthium strumarium (Xanthium sibiricum P.), Lepidium didymum,* or any combination thereof.

A second aspect the present invention relates to a plant, plant cell, tissue, organ, material or seed obtainable by a method according to the above aspects and embodiments, wherein the color tag is introduced by labelling the seed with a chemical color tag or by introducing or inducing a genomic color tag using a genome modification system.

In one embodiment of the second aspect of to the present invention the plant, plant cell, tissue, organ, or seed is selected from a plant originating from a genus selected from the group consisting of *Hordeum, Sorghum, Saccharum, Zea, Setaria, Oryza, Triticum, Secale, Triticale, Malus, Brachypodium, Aegilops, Daucus, Beta, Eucalyptus, Nicotiana, Solanum, Coffea, Vitis, Erythrante, Genlisea, Cucumis, Marus, Arabidopsis, Crucihimalaya, Cardamine, Lepidium, Capsella, Olmarabidopsis, Arabis, Brassica, Eruca, Raphanus, Citrus, Jatropha, Populus, Medicago, Cicer, Cajanus, Phaseolus, Glycine, Gossypium, Astragalus, Lotus, Torenia, Allium, Spinacia or Helianthus,* preferably, the plant or plant cell originates from a species selected from the group consisting of *Hordeum vulgare, Hordeum bulbusom, Sorghum bicolor, Saccharum officinarium, Zea spp., including Zea mays, Setaria italica, Oryza minuta, Oryza sativa, Oryza australiensis, Oryza alta, Triticum aestivum, Triticum durum, Secale cereale,* Triticale, *Malus domestica, Brachypodium distachyon, Hordeum marinum, Aegilops tauschii, Daucus glochidiatus, Beta spp.,* including *Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Allium tuberosum, Helianthus annuus, Helianthus tuberosus* and/or *Spinacia oleracea.*

In a third aspect the present invention relates to a method of distinguishing at least one plant comprising at least one active color tag from potentially present undesired weeds, wherein the method comprises the following steps: (i) providing at least one plant that comprises at least one active color tag as defined in any one of the above aspects and/or embodiments, and further providing at least one platform, preferably a mobile platform, more preferably an autonomous mobile platform, equipped with at least one cognate sensor as defined herein for embodiments related to the sensor; (ii) optionally: irradiating the at least one plant with light; (iii) capturing image data of the at least one plant comprising the at least one active color tag using the at least one cognate sensor; (iv) processing the image data; (v) distinguishing at plant comprising the active color tag from potentially present undesired weeds.

In one embodiment of the third aspect of the present invention, a plant of interest is distinguished from potentially present undesired weed by combining the detection of a color signal with the analysis of the leaf shape, e.g. with an RGB camera. Thus, the sensor-based detection can be based on the color tag as well as the leaf shape, which can result in a very precise and controlled system by integration of two methods.

In a preferred embodiment of the third aspect of the present invention, the method comprises the additional step of (vi) of eliminating potentially present undesired weeds.

It is an object of the present invention to provide a method together with a data acquisition and evaluation system for performing data analysis for plant identification based on the detection of an active color tag in a field by a platform, preferably a mobile platform, which enables a reliable and/or time-efficient differentiation of desired plants and potential weeds.

The method preferably comprises a step of using a selected computer algorithm, optionally assisted by any means of artificial intelligence (Al). It is particularly preferred that the computer algorithm processes the captured analysis data set by accessing and/or analyzing a database which preferably comprises reference data sets to be able to determine one or more plants. Therefore, the computer algorithm and/or AI is preferably adapted to consider the analysis data set in relation to at least one reference data set, which are stored in the database. The algorithm and/or AI distinguished the desired plant from weeds via the presence of a color signal. Thus, the at least one reference data set contains information on the color signal caused by a particular color tag. When the distinction between desired plant and undesired weed is based on both the presence of a color signal and the leaf shape, the reference data set should also contain information on the leaf shapes of the desired plant or plants and preferentially of potential undesired weeds. Thereby the algorithm and/or AI can assign one or more detected plants to the group of desired plants or the group of undesired weeds based on the presence or absence of the color signal. An algorithm and/or AI according to the present invention can compare the captured analysis data set with one or more data sets comprising information on more than one color tag and the respective color signal. Thus the algorithm and/or AI may be capable to assign different plants harboring different color signals from different color tags to the group of desired plants.

In particular, it is preferred that the cognate sensor sensors are attached at the underside of a platform, wherein the platform is preferably a mobile platform. A mobile platform is in particular horizontally moveable in parallel of the field ground, wherein its underside facing the field ground. This embodiment has the advantage that data can be captured while moving the mobile platform above the field.

A mobile platform is a ground-based device and/or an aerial device, preferably an autonomous mobile platform. Preferably, an autonomous mobile platform is an unmanned mobile platform and/or can be remote-controlled. In particular, the mobile platform can be moved horizontally in parallel of the field ground, preferably at an altitude up to 0.5 m, 1 m, 2 m, 3 m, 5 m, 10 m above the plants. The ground-based device can be for example a tractor or an automat. An example of the aerial device is a drone.

One or more, possibly different, platforms, preferentially mobile platforms, e.g. a ground-based mobile device and an aerial mobile device, me be combined to form a robotic system. It is also possible to upgrade existing agricultural machinery, such as tractors, which may also function as parts of a robotic system together with other platforms. In a robotic system it can be envisaged that different functions, e.g. detection of weeds and elimination of weeds, may be performed by different platforms.

A mobile platform according the present invention preferentially comprises inertial measurement unit. The inertial measurement unit is preferably a spatial combination of several inertial sensors for motion detection, e.g. accelerate sensors and rotational speed sensors. The inertial measurement unit can also be used for stabilization of the mobile platform. Preferably the mobile platform additionally comprises a system for geopositioning.

The preferentially mobile platform is preferred to be a semi-autonomous or most preferentially an autonomous mobile platform. The method preferably comprises a motion schedule for the autonomous mobile platform. This provides an automatic motion of the autonomous mobile platform. The motion schedule provides preferably that one motion at one timepoint is sufficient for capturing all necessary data. In particular, the mobile platform can be automatically controlled on the basis of the previously created motion schedule. Therefore, the mobile platform can be preferably connected with a control unit for controlling the movement of the mobile platform. It is further preferred, that motion data of an actual movement of the mobile platform can be captured using the inertial measurement unit and/or the geopositioning data during the motion of the mobile platform in accordance with the motion schedule. Actual movement data and the data of the motion schedule can differ, e.g. due to environmental conditions and such discrepancies may be corrected. Further, the control unit can be preferably adapted for controlling the mobile platform. Particularly preferred, a memory unit can be connected with the controlling unit and the mobile platform for storing and accessing the motion schedule.

Preferably, the one or more (mobile) platforms are connected to a main server. It is further preferred that the main server can be adapted for processing captured data. The main server can be preferably located at a main location of administration. The main server can comprise a memory unit for storage of captured and/or processed data and particularly preferred a control unit for processing captured data. The main server preferentially comprises a control unit, which can be preferably adapted for controlling the mobile platform. Particularly preferred, the memory unit can be connected with the controlling unit and the mobile platform for storing and accessing the motion schedule.

Particularly preferred, the method comprises processing the data on the mobile platform and/or the main server during the operating process.

Preferably, the mobile platform and/or the main server comprise a memory unit for storing captured data and/or processed data and/or the motion schedule and/or field plan information. The field plan information may comprise field information for defining field locations and field dimensions, in particular field piece information for defining field piece locations and field piece dimensions. It is preferred that field pieces form a grid of the field.

In a preferred embodiment, the captured data and/or the processed data are transferred between the mobile platform and the main server and/or via a wire- / cable-bound connection and/or a wireless connection using any available standard or technique.

It is preferred that the platform comprises a memory unit for storing captured data. Preferably, the main server can download said captured data for processing. Therefore, the mobile platform and/or the main server comprise an interface, in particular for data transmission, preferably wireless data transmission.

Weeding may comprise chemical and/or mechanical weed control. The weeding may thus be performed by targeted herbicide use or by any means that remove undesired weed from the vicinity of the desired plant, destroys or kills undesired said weed or hinders, preferably abolishes, the growth of said undesired weed. Different weeding methods in the context of weeding platforms are known to the skilled person.

In a fourth aspect the invention relates to a use of an active color tag comprised by at least one plant, plant cell, tissue, organ, seed, or material, preferably during an early growth stage up to the 4-leaf-stage, for automated robot assisted weeding.

In one embodiment of the fourth aspect of the present invention, the method combines the detection of a color signal with the analysis of the leaf shape, e.g. with an RGB camera. Thus, the sensor-based detection can be based on the color tag as well as the leaf shape, which can result in a very precise and controlled system by integration of two methods.

In one embodiment of the fourth aspect of the present invention, the color tag is a chemical color tag, preferably wherein the chemical color tag consists of or comprises a dye being selected from the group of a simple coumarin, including scopoletin or esculetin, more preferably wherein the chemical color tag consists of or comprises a dye being coupled to glucose, including esculin or scopolin.

In another embodiment of the fourth aspect of the present invention, the color tag is a genomic color tag introduced by a genome modification system by (a) introducing at least one insertion nucleotide sequence, wherein the expression of said insertion nucleotide sequence causes an altered level of at least one compound detectable by at least one cognate sensor, wherein the at least one insertion nucleotide sequence is a plant endogenous sequence, or by (b) modifying or introducing at least one regulatory region that causes an altered expression of an endogenous nucleotide sequence, wherein the altered expression of said endogenous nucleotide sequence causes an altered level of at least one compound detectable by at least one cognate sensor, or any combination of (a) and (b).

The present invention will now be further described based on the following, non-limiting, Examples.

### Examples

### Example 1:

For the experiment, four solutions with different concentrations of esculin hydrate (Sigma-Aldrich Chemie GmbH, Taufkirchen, Germany) were used. The concentrations of the solutions were 0.1 mM, 1 mM, 10 mM and 30 mM as well as a negative control (water). For each concentration, two sets (1 set = 200 g = 200.000 seeds fit in 1 liter of water) of sugar beet seeds of two different varieties (Leopolda and Daphna) were kept for 24 hours in the different concentrations of esculin hydrate dissolved in 1l of water. Eventually, seeds were dried and planted into 4 boxes (200 seeds per box) of turf-sand mixtures. Afterwards, the boxes were covered and kept at room-temperature to control first germination. After two days, boxes were uncovered and illuminated with sunlight, respectively. Seedlings were collected as soon as plants germinated. Esculin fluorescence in seedlings was following analyzed under a fluorescence stereoscope (UV = 360 nm, pictures taken at an exposition to light of 500 nm) every day for two days.

The germination of seedlings started one day after exposure to sun light. Blue fluorescence color was visible in the seedlings on the day of germination under a fluorescence stereoscope. It appeared in the hypocotyl at a higher rate than in the cotyledons. The blue fluorescence color of the molecule is clearly different from the red auto-fluorescence of chlorophyll (Fig. 2). However, the blue fluorescence color almost disappeared on the third day after germination and only the red auto-fluorescence color of chlorophyll was detectable. (Fig. 3) The observations also revealed that higher concentrations of esculin did not lead to a certain insensitivity of the esculin fluorescence staining and/or higher number of stained seeds, respectively. Furthermore, the experiment showed that esculin hydrate is a molecule which is able to pass the testa of seeds and enters the embryo. Moreover, esculin hydrate is kept in the seedlings when they germinate. The experiment further showed that seedlings tagged with esculin hydrate are visible under a fluorescence stereoscope.

### Example 2:

As detailed above, various additional options suitable for seed labelling were planned and initially tested. For example, seeds can be labelled with dye solutions comprising different concentrations of the dye (e.g. scopolin or scopoletin) for different periods of time, such as 12-48 hours. Alternatively the seeds can be coated, wherein the seed coating comprises the dye. After germination, the color signals can be analyzed. For Scopolin or Scopoletin the fluorescent color signal can for instance be assessed using a high intensity UV inspection lamp and macroscopic analysis. The color signal can be analyzed over several days after germination to determine how long a color signal is visible for each preparation method.

### Example 3:

A genomic color tag can be realized by several different means. One example is to use an insertion nucleotide according to the present invention. Such an insertion nucleotide could comprise the nucleic acid sequence encoding an enzyme or a cofactor of an enzyme or a different factor that influences the activity of an enzyme. The enzyme that is expressed or influenced in this manner would be an enzyme in the synthesis pathway of a dye, e.g. it may be composed of or comprise a fluorophore or chromophore. Said dye may be an intermediate in a synthesis pathway. The enzyme of choice may be the enzyme that directly converts a precursor into the dye or, for instance, an enzyme that catalyzes a rate limiting step in the synthesis pathway. In some cases it may be necessary to introduce and/or affect (by introduction of a cofactor or influencing factors) two or more enzymes, in one synthesis pathway. The insertion nucleotide would in the simplest case comprise all necessary elements for transcription and translation. Thus, is would comprise e.g. a promoter and a transcription terminator, operably linked to the coding sequence. The promoter sequence is of particular importance to allow expression in the correct part of the plant, such as the cotyledon, hypocotyl or otherwise in the canopy of the plant, and in the desired developmental stage, i.e. at least in the early growth phase. The enzyme or cofactor or influencing factor introduced as described herein may be naturally existing in the plant into which the color tag is to be introduced, i.e. the plant of interest. An introduced nucleotide sequence may thus, for instance, be an additional copy of a nucleotide sequence encoding such an enzyme or factor but e.g. with a different promoter as described above. The enzyme or factor could also be an ortholog or a structurally or functionally similar enzyme or factor from a different plant species. It can also be envisaged that the enzyme or factor introduced by means of an insertion nucleotide as described herein exists in a different plant species and has no ortholog or a structurally or functionally similar enzyme or factor in the plant of interest. In the latter case, an enzyme could catalyze or facilitate a reaction that exists in a synthesis pathway of the plant of interest; or it may catalyze or facilitate a reaction that does not naturally exist in the plant of interest, synthesizing either a dye that naturally occurs in the plant of interest (but is naturally synthetizes differently) or dye that is a compound or molecule that only occurs naturally in a different plant species but not in the plant of interest. For the synthesis of a dye it may be required to introduce or affect two or more enzymes, using two or more insertion nucleotides.

It may be of particular interest to use a transient color tag. With a transient color tag it can be ensured that the dye or the expressed enzyme(s) or factor(s) is/are not present, or not present in an elevated amount and/or concentration, in the grown plant and/or the harvested product. A transient color tag generally decreases a possible influence of the dye itself or expression of the nucleotide sequence(s) encoding the enzyme(s) or factor(s) used in the color tag on the plant. Thus, it is ideal to use a promoterthat is not constitutively active but rather a promoter that does not cause expression in later developmental stages close to harvesting. Ideally a promoter that is only active during the early growth phase is used.

### Example 4:

A different approach to realize a genomic colortag is by modifying or inserting a regulatory region of interest. The most straightforward regulatory regions to insert or modify are promoter regions. In the synthesis pathway of a dye, the promoter regulating the expression of a nucleotide sequence encoding an Enzyme, or a cofactor or influencing factor of such an enzyme, could be exchanged by a different promoter or it could be modified, for instance in the TATA-Box, to alter the expression. By increasing the expression of a nucleotide encoding such an enzyme or cofactor or influencing factor, at least in the early growth stage and at least in the relevant parts of the plant, such as the cotyledon, hypocothyl or otherwise in the canopy, the concentration of a dye rises, wherein the increased concentration of said dye yields a detectable color signal. Similar to insertion nucleotides, the enzyme of choice, which has a changed expression of its encoding nucleotide sequence itself or is influenced by the changed expression of a nucleotide sequence encoding a cofactor or influencing factor, may be the enzyme that directly converts a precursor into the dye or, for instance, an enzyme that catalyzes a rate limiting step in the synthesis pathway (for coumarins, the target enzyme may be F6'H1; see Shimizu Front, Plant Sci. 2014 Nov 3;5:549). In some cases it may be necessary to alter the expression of two or more nucleotides encoding two or more enzymes and/or or cofactors and/or influencing factors of enzymes, in one synthesis pathway.

A dye might be an intermediate of a synthesis pathway or may generally be subject to conversion into a different compound by further enzymes. Thus, the modification or exchange of a promoter region of a nucleotide sequence encoding an enzyme or cofactor or influencing factor of an enzyme, may be used to reduce the production or activity of an enzyme that converts a dye into a different molecule or compound. The reduction of production or activity of such an enzyme, which reduces the concentration of the dye, can be combined with the increased production or activity (as described herein using a insertion nucleotide sequence and/or the modification or introduction of a regulatory sequence) of one or more enzymes that increase the concentration of the dye. The reduced production or activity of an enzyme reducing the concentration of a dye may also be used when the compound is applied as a chemical color tag, wherein the compound or precursor may also be applied directly or is produced by a seed-associated microbe. Generally, the activity of an enzyme might be reduced by reducing the expression of nucleotide sequences encoding activating or promoting factors, or increased by an increasing the expression of nucleotide sequences encoding of inhibiting factors.

When an existing promoter is exchanged to alter the expression of that nucleotide sequence of interest, the choice of the promoter that is introduced is critical. Ideally, the choice of promoters introduces a transient color tag.

## Claims

1. A method for labelling a plant, plant cell, tissue, organ, seed, or material with at least one color tag active at least during an early growth stage of a plant and allowing the discrimination of desired plants from undesired weeds by means of color signals, wherein the at least one color tag is detectable by at least one cognate sensor, the method comprising:
(i) providing at least one plant cell, or at least one plant, tissue, organ, seed, or material comprising the same;
(ii) introducing or inducing at least one color tag into/in the at least one plant cell, or into the at least one plant, tissue, organ, seed, or material comprising the same;
(iii) optionally: selecting at least one plant cell, or at least one plant, tissue, organ, seed, or material comprising the same, wherein the selection is based on the successful integration of the color tag;
(iv) obtaining at least one plant cell, or at least one plant, tissue, organ, seed, or material comprising the same, that comprises the color tag, so that the color tag is active during the early growth stage of a plant; and
(v) optionally: seeding the plant seed and discriminating the desired plants comprising an active color tag from undesired weeds;
wherein the color tag is introduced in a transient way, or wherein the color tag is a natural color tag, or any combination thereof.

2. The method according to claim 1, wherein the color tag is introduced by labelling a plant seed with a chemical color tag, or by introducing or inducing a genomic color tag, or any combination thereof.

3. The method according to claim 1 or claim 2, wherein the early growth stage is the time period up to the 4-leaf-stage.

4. The method according to claim 2, wherein the color tag is a chemical color tag, preferably wherein the chemical color tag consists of or comprises a dye being selected from the group of a coumarin, including scopoletin or esculetin, more preferably wherein the chemical color tag consists of or comprises a dye being coupled to glucose, including esculin or scopolin.

5. The method according to claim 2, wherein the color tag is a genomic color tag introduced by a genome modification system by
(a) introducing at least one insertion nucleotide sequence, wherein the expression of said insertion nucleotide sequence causes an altered level of at least one compound detectable by at least one cognate sensor, wherein the at least one insertion nucleotide sequence is a plant endogenous sequence, or by
(b) modifying or introducing at least one regulatory region that causes an altered expression of an endogenous nucleotide sequence, wherein the altered expression of said endogenous nucleotide sequence causes an altered level of at least one compound detectable by at least one cognate sensor,
or any combination of (a) and (b).

6. The method according to claim 5, wherein the genome modification system comprises at least one site-directed nuclease, nickase or an inactivated nuclease, preferably a nucleic acid guided nuclease, nickase or an inactivated nuclease, or a sequence encoding the same, and optionally at least one guide molecule, or a sequence encoding the same.

7. The method according to any of the preceding claims, wherein the at least one cognate sensor is an image sensor, optionally included in a camera, wherein the image sensor is an RGB sensor, a multispectral sensor, a hyperspectral sensor, a fluorescence sensor, or a monochrome sensor, or a combination of at least two sensors, wherein the at least one cognate sensor optionally comprises a modulated irradiation source for irradiating at least one plant, plant cell, tissue, organ, seed, or material with light, wherein said modulated irradiation source is preferably used in combination with the image sensor.

8. A plant, plant cell, tissue, organ, material or seed obtainable by a method according to any of the preceding claims, wherein the color tag is introduced by labelling the seed with a chemical color tag or by introducing or inducing a genomic color tag using a genome modification system.

9. The plant, plant cell, tissue, organ, or seed according to claim 8, wherein the plant, plant cell, tissue, organ, or seed is selected from a plant originating from a genus selected from the group consisting of *Hordeum, Sorghum, Saccharum, Zea, Setaria, Oryza, Triticum, Secale, Triticale, Malus, Brachypodium, Aegilops, Daucus, Beta, Eucalyptus, Nicotiana, Solanum, Coffea, Vitis, Erythrante, Genlisea, Cucumis, Marus, Arabidopsis, Crucihimalaya, Cardamine, Lepidium, Capsella, Olmarabidopsis, Arabis, Brassica, Eruca, Raphanus, Citrus, Jatropha, Populus, Medicago, Cicer, Cajanus, Phaseolus, Glycine, Gossypium, Astragalus, Lotus, Torenia, Allium, Spinacia or Helianthus,* preferably, the plant or plant cell originates from a species selected from the group consisting of *Hordeum vulgare, Hordeum bulbusom, Sorghum bicolor, Saccharum officinarium, Zea spp., including Zea mays, Setaria italica, Oryza minuta, Oryza sativa, Oryza australiensis, Oryza alta, Triticum aestivum, Triticum durum, Secale cereale,* Triticale, *Malus domestica, Brachypodium distachyon, Hordeum marinum, Aegilops tauschii, Daucus glochidiatus, Beta spp.,* including *Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Allium tuberosum, Helianthus annuus, Helianthus tuberosus* and/or *Spinacia oleracea.*

10. The method according to any of claims 1 to 7, wherein the at least one undesired weed is selected from the group consisting of *Alopecures myosuroides, Acalypha australis L., Amaranthus retroflexus, Agropyron repens (L) Beauv., Agrostis gigantea Roth. Fl. Germ, Brassica napus, Amaranthus blitoide, Amaranthus lividus Loisel., Amaranthus palmerii, Amaranthus patulus bertoloni, Amaranthus powellii, Capsella bursa-pastoris, Ambrosia artemisiifolia, Ambrosia trifida, Amethystea caerulea L., Anthemis arvensis L., Anthemis cotula L., Artemisia scoparia, Artemisia sieversiana, Chenopodium album, Fumaria officinalis, Galeopsis tetrahit, Barbarea vulgaris R.Br., Bidens tripartite, Galium aparine, Calystegia hederacea Wall, Calystegia sepium, Geranium spp., Cenchrus echinatus, Centaurea cyanus, Lamium purpureum, Chenopodium glaucum L., Cheno-podium serotinum L., Matricaria camomilla, Cirsuim vulgare, Commelina communis L., Conium maculatum, Matricaria inodora, Cuscuta campestris (pentago-na), Cuscuta pentagona Engelm., Cyperus esculentus, Datura stramonium L., Digitaria sanguinalis, Digitaria violascens Link, Mercurialis annual, Echinochloa phyllopogon Stapf, Eleusine inica(L) Gaertn., Elsholtzia ciliata (Thunb.) Hylander, Poa Annua, Polygonum aviculare, Erigeron annuus L., Erigeron canadensis L., Erysimum cheiranthoides L., Fallopia japonica, Polygonum convolvu-lus, Polygonum persicaria, Galinsoga parviflora, Senecio vulgaris, Solanum nigrum, Helianthus annuus, Hibiscus trionum, Hordeum vulgare, Iva annua, Kochia scoparia, Stellaria media, Thlaspi arvense, Veronica arvensis, Viola arvensis, Abutilon theophrasti, Aethusa cynapium, Myosoton aquaticum (L.) Moench, Oxalis corniculata L., Panicum capillare, Panicum dichotomiflorum, Persicaria convolvulus L., Persicaria lapathifolia (L.) S.F.Gray, Persicaria longiseta (De Bruyn) Kitag., Persicaria nepalensis (Meisn.) H.Gross, Persicaria vulgaris Webb et Moq., Phragmites australis (Cav.) Trin. Ex Steud., Atriplex patula, Cirsium arvense* (*Cirsium Segetum* / *Cephalonoplos segetum), Echinochloa crus-galii, Polygonum bungeanum T., Elymus repens* / *Elytrigia repens, Polygonum lapatifolium, Polygonum pensylvaticum, Myosotis arvensis, Portulaca oleracea, Rhaponticum repens, Rorippa atrovirens Ohwi et Hara, Rorippa islandica (Oeder) Borlas, Rorippa sylvestris Besser, Rumex acetosella L., Rumex crispus L., Rumex japonicus Houtt., Rumex longifolius DC., Rumex obtusifolius L., Sagina japonica (Sw.) Ohwi, Salsola tragus, Salvia reflexa, Sonchus arvensis (brachyotus DC), Setaria glauca, Setaria viridis, Atriplex hastate, Avena fatua, Solanum ptycanthum, Solanum rostratum, Solanum sarrachoides, Convolvulus arvensis, Equisetum arvense, Sonchus brachyotis DC., Spergula arvensis L., Malva spp., Stellaria alsine Grimm var.undulata (Thunb.) Ohwi ,Polygonum amphibium, Symphytum officinale L., Taraxacum (officinale and mongolicum) ,Sinapis arvensis, Solanum tuberosum, Stachys arvensis, Veronica hederifolia L., Veronica persica, Urtica urens, Xanthium strumarium (Xanthium sibiricum P.), Lepidium didymum,* or any combination thereof.

11. A method of distinguishing at least one plant comprising at least one active color tag from potentially present undesired weeds, wherein the method comprises the following steps:
(i) providing at least one plant that comprises at least one active color tag as defined in any one of claims 1, 2, 4 or 5, and further providing at least one platform, preferably a mobile platform, more preferably an autonomous mobile platform, equipped with at least one cognate sensor as defined in claim 7;
(ii) optionally: irradiating the at least one plant comprising the at least one active color tag with light;
(iii) capturing image data of the at least one plant comprising the at least one active color tag using the at least one cognate sensor;
(iv) processing the image data;
(v) distinguishing at plant comprising the active color tag from potentially present undesired weeds.

12. The method of claim 11, comprising an additional step (vi) of eliminating potentially present undesired weeds.

13. A use of an active color tag comprised by at least one plant, plant cell, tissue, organ, seed, or material, preferably during an early growth stage up to the 4-leaf-stage, for automated robot assisted weeding.

14. The use of an active color tag according to claim 13, wherein the color tag is a chemical color tag, wherein the chemical color tag consists of or comprises a dye being selected from the group of a coumarin, including scopoletin or esculetin, more preferably wherein the chemical color tag consists of or comprises a dye being coupled to glucose, including esculin or scopolin.

15. The use of an active color tag according to claim 13, wherein the color tag is a genomic color tag introduced by a genome modification system by
(a) introducing at least one insertion nucleotide sequence, wherein the expression of said insertion nucleotide sequence causes an altered level of at least one compound detectable by at least one cognate sensor, wherein the at least one insertion nucleotide sequence is a plant endogenous sequence, or by
(b) modifying or introducing at least one regulatory region that causes an altered expression of an endogenous nucleotide sequence, wherein the altered expression of said endogenous nucleotide sequence causes an altered level of at least one compound detectable by at least one cognate sensor,
or any combination of (a) and (b); preferably wherein the genome modification system comprises at least one site-directed nuclease, nickase or an inactivated nuclease, preferably a nucleic acid guided nuclease, nickase or an inactivated nuclease, or a sequence encoding the same, and optionally at least one guide molecule, or a sequence encoding the same.
